# EUROPEAN PATENT SPECIFICATION

(11) **EP 2 211 982 B1**
(45) Date of publication and mention of the grant of the patent: **06.04.2016**
(21) Application number: 08801392.5
(22) Date of filing: 10.10.2008
(51) Int. Cl.: A61N 1/32

(54) **AN ELECTROPORATION DEVICE FOR IMPROVED ELECTRICAL FIELD CONTROL**
ELEKTROPORATIONSVORRICHTUNG FÜR VERBESSERTE ELEKTROFELD-KONTROLLE
DISPOSITIF D'ÉLECTROPORATION POUR UNE COMMANDE DE CHAMP ÉLECTRIQUE AMÉLIORÉE

(30) Priority: 11.10.2007 US 979211 P
(43) Date of publication of application: 04.08.2010
(73) Proprietor: Region Hovedstaden v/Herlev Hospital, 2730 Herlev (DK)
(72) Inventor: GEHL, Karen, Julie, 2720 Vanlose (DK); STAAL, Lasse, Guldborg, 4040 Jyllinge (DK)
(74) Representative: Plougmann Vingtoft a/s
(86) International application number: PCT/DK2008/000358
(87) International publication number: WO 2009/046720

(56) References cited:
- WO-A-00/23143
- US-A1- 2002 151 866
- US-A1- 2005 215 941
- US-A1- 2007 043 397

## Description

### Field of the invention

The present invention concerns a device for electroporation, in general and more specifically the present invention concerns a device for administering therapeutic molecules, such as a drug, an isotope or genetic material, enhanced by electric pulses causing electroporation of and/or electrophoretic effects in a target region of a patient's body. More particularly the invention relates to a device wherein a plurality of electrodes/electrotherapeutic devices are inserted into or into the vicinity of a target tissue for applying an electrical field for opening cell membranes in that tissue, and where a dose of therapeutic molecules is administered to that target tissue.
More specifically the invention relates to a device for performing electroporation in deeper-lying tissues of the body of a patient. More specifically the invention relates to a device and a method that may be applied for electroporation in tissues of the central nervous system, particularly the brain.

### Background of the invention

In the treatment of diseases in the brain, e.g. brain cancer, as well as diseases in other anatomical areas of a body, physical access to a diseased tissue region may be a challenge. This is especially the case if the diseased region lies deep within the body of the patient. Furthermore, efficient delivery and subsequent uptake of therapeutic molecules, such as a drug or genetic compound, to an anatomical target tissue is often a problem.
Electroporation is a known method used to deliver drugs and genetic material to various biologic tissues, where the uptake of these substances into tissue cells is enhanced through the application of electric pulses of specific amplitudes. The delivery of chemotherapeutic agents by electroporation is also known as electrochemotherapy (ECT) and the delivery of genes as Electro Gene Transfer (EGT). In ECT and EGT applications, electroporation is used to create a transient permeabilization of the cell membranes in a target tissue area with the purpose of enhancing the uptake of the chemotherapeutic agents as well as the uptake and expression of genetic materials.

In order to provide an efficient electroporation, two or more electrode poles have to be brought into - or into close vicinity of - the region to be treated (target region). Examples of devices used for Electroporation are known from US 5 674 267 , US 2002/0151866 and US 6 278 895. These devices consist of an array of pointed and/or sharpened conductive needle-type electrodes arranged as individual electrodes that are held together by, and inserted via, some external plate-shaped element providing a fixed distance between, and relative position of, the individual needles. If the target region is situated in a remote region of the body, such as the deeper regions of the brain, the insertion and placement of the multiple individual pointed and/or sharpened electrodes may in itself create cutting and/or displacement-related trauma to intervening tissue through which the electrodes need to traverse in order to be located in the desired region.
Furthermore, maintaining control with the paths of individual needle-type electrodes as they traverse intervening tissues that may have different morphologies is a challenge that increases with the length of insertion. The distance between electrodes is a critical parameter in the creation of an electric field with desired characteristics, and the possible, uncontrolled deviations from desired paths that may result from deep insertions may substantially affect field homogeneity and resulting drug uptake.
Yet further, a large access area must be available for the insertion of said arrays, and specifically for applications in the brain this will entail creating an excessively large hole in the patient's skull.
Therefore, it is evident that the mentioned prior art devices are only well-suited for treatment in target regions in close proximity to an outer surface of the body, because an attempt to treat deeper-lying regions would cause excessive trauma to the intervening tissue.
The application of electric energies to tissue with the intention of obtaining specific medical effects is an already known and described method for various indications. Deep-brain stimulation, cardioversion, neural prostheses and radio-frequency ablation are all examples of applications of electric energies to obtain specific effects.

Typically, transfer of electric energies to tissue happens through one or more electrode applicators that are placed in or against the tissue that is to receive the energy.

For these and other applications, an important goal is to provide the best possible transfer of energy under the circumstances. This is especially an issue in minimally invasive approaches, where there is often a trade-off between the desire for the largest possible transmitting surface and the constraints that are imposed by small working spaces and the desire to minimize damage to intervening and adjacent tissue during applicator insertion.

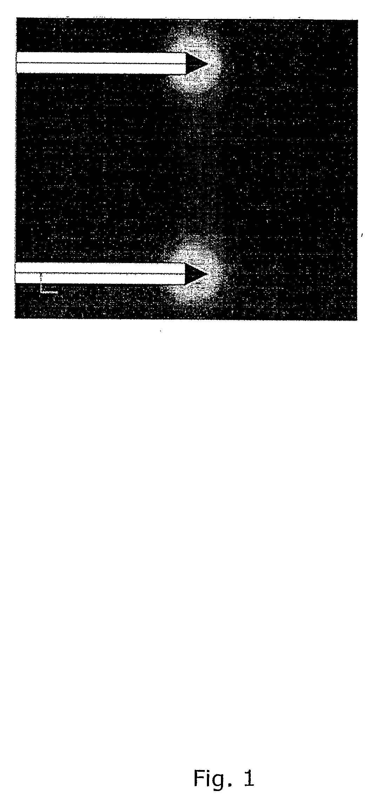

Needle-shaped electrode applicators are frequently used as a means of energy transfer. Such electrode applicators are well-suited to subcutaneous as well as percutaneous applications since needles will easily cut through tissue during placement of the electrodes. However, needles and other pointed electrode configurations are characterized by a pronounced tendency to produce field peaks (areas of very strong field gradients) - the so-called hot-spots - at points and other sections. Such hot-spots are illustrated in figure 1, where areas of elevated field strength are shown as thickened colorations near the electrode point and the angular shoulders of the point. These hot-spots are associated with local cell death - i.e. necrosis - when they occur in tissue that is to be treated, and are detrimental to the effects of some treatments especially in the field of electroporation.

Another solution frequently applied is conductive plates. While this approach is well suited to surface applications, the use of plates becomes problematic for minimally invasive applications where the placement of plates is frequently a challenge, and where excessive damage may be inflicted to intervening tissue.

A primary area of application of electrical energies is within the field of electroporation. Electroporation is a well-known method for creating transient and non-transient permeabilization of living cells in vitro and in vivo by application of an electric field of a certain magnitude. Such permeabilization may for instance be useful in the transfer of substances such as chemotherapeutic agents or genetic compounds across cell membranes to reach the interior of cells.

For in vivo applications, several invasive electrode applicators have been developed. These are meant for percutaneous insertion into the tissue to be treated, and comprise electrode transmitters shaped as needles.

Such needle electrodes provide reasonably efficient transfer of an electric field to skin or tissue residing immediately below the skin surface. For these applications, the creation of hot-spots - with resulting tissue necrosis in affected areas - is less problematic. However, for applications to sensitive tissues - such as neurological or cardiac tissues - hot-spots and resulting tissue necrosis may result in the loss of important tissue functions. There is therefore a need for electrode transmitters for in vivo applications that are configured to minimize the creation of hot-spots.

Another unaddressed issue with needle electrode applicators is the cutting or piercing of tissue that takes place during electrode applicator insertion. While this is considered unproblematic for the treatment of skin or tissue residing immediately below the skin surface, there is a need for less-traumatizing electrode transmitter types for more sensitive tissue areas such as neurological and cardiac tissue.

Yet another unaddressed issue with currently available electrode applicators is the lack of control over field distribution. Currently available electrode transmitters are conductive along at least an elongate portion of their entire free length, which means that all tissue, that are in contact with this elongate portion will be affected by the electric field. This is less problematic in treatments that are meant to target the skin or tissue residing immediately below the skin, but becomes problematic once an operator desires to treat deeper-lying tissue regions. Accordingly, there is a need for electrode transmitters that applies an effective, controllable electrical field for electroporation purposes without affecting surrounding or intervening layers of tissue

A related issue regards the ability of currently available electrode applicators to generate precisely defined, three-dimensional electric fields that may be configured to conform to the three-dimensional contours of a particular tissue region - e.g. a tumour. Currently available electrode applicators, as for instance disclosed in US 6,278,895, provide needle electrodes that have distal pointed ends disposed in a single plane. Such single-plane electrode applicators are less well suited to the generation of complex, potentially irregular and possibly three-dimensional fields that may be optimal for minimally invasive applications.

Other needle applicators, as for instance disclosed in US 5,439,440 may provide needle electrodes that may be adjusted in depth to position distal ends in multiple planes. While such a configuration is perceived as superior in providing fields that may conform to individual lesion anatomies, parameters of the field that is applied to the target tissue are still severely affected by the pointed shapes of electrode distal ends and especially by the positions of said electrodes and their distal ends relative to one another. For instance, it is evident that the shape and gradient distribution of a field that is generated between two needle electrodes with pointed tips will change as the depth of insertion of one electrode is changed relative to the depth of insertion of the other electrode. More specifically, the parameters of a field that is generated between a first pointed electrode and a second pointed electrode will differ, depending on whether the primary area of field transmission is the shaft of the respective electrode, the shoulders of the pointed distal end, or the distal tip itself.

Accordingly there is a need for electrode transmitters that permit the generation of complex, potentially irregular field shapes while providing field parameters that are not influenced by the relative positions of electrode distal ends.

Another issue that is related to the pointed shape of the distal ends of currently available electrode applicators is the lack of transmission surface scalability that is associated with this particular shape. It is possible to increase the diameters of currently available applicators, thereby providing larger surface areas to facilitate improved transfer of electrical currents to tissue. However, the problematic hot-spot effect remains due to the constant geometry of a pointed tip. This limits the ability of currently available electrode applicators to transfer electric fields at their distal ends. Accordingly the inventors realize that there is a need for electrode applicators that obtain more efficient distal transfer of electric fields, and preferably without increasing the dimensions of the applicators.
Devices for electroporation are known in the art. Such devices often have superficial similarities with devices used in the field of thermal ablation, e.g. RF ablation. However, the functional principles are very different. In the field of thermal ablation, the destruction of cells is accomplished through the application of intense thermal energy, whether by application of focused ultrasound, RF energy, microwaves, laser, cryogens or otherwise.
While cell death may be one ultimate objective of electroporation (e.g. electrochemotherapy), electroporation is a strictly non-thermal process relying on pore formation in target tissue cell membranes through the application of a precisely calculated electrical field with specific amplitude and duration. The purpose is to promote the transportation of otherwise non-permeating or less-permeating biologically active molecules into the cells by transient permeabilization, either to provide a treatment of the cell or to destroy it. Contrarily, in the field of ablation, thermodynamics are exploited to create a gradually expanding area of cell death through the aggressive application of thermal energy, The purpose is to promote cell death by complete and irreversible disruption of cell membranes.
Electroporation is a science of thresholds, and the ultimate goal is to precisely and reliably generate a field that may promote uniform uptake of biologically active molecules in a target tissue. In this process, burning/scarring of target and/or adjacent tissue is strongly undesirable, since it may interfere with the uptake of molecules through changes in tissue conductivity.

### Object of the invention

There is thus a need for an electroporation device and an electroporation method that overcomes the shortcomings of the presently known devices and methods. It is an object of the present invention to provide such a device It is a further object of the invention to provide an electroporation device which can be manoeuvred to deeper-lying regions of the body or to regions that are otherwise difficult to access, and to do so with the least amount of injury to the tissue. E.g. for applications in the brain, it is an objective to provide a device requiring the smallest possible entry hole while providing the largest possible electric field. A further object of the invention is to provide an electroporation device capable of delivering an improved, flexible and more efficient electric field in order to enhance the transfer of e.g. drugs, isotopes, genetic materials or other therapeutic molecules through cell membranes of a target tissue/region. By providing an improved, more efficient and more readily controlled electrical field, the energy applied through electrodes to the tissue may be reduced. Thereby, unintended damages to the tissue, especially the tissue immediately surrounding the electrodes may be reduced.

An objective of the present invention is therefore to provide an electrode tip/electrotherapeutic device tip design that is configured to minimize the creation of hot-spots in living tissue during the application of electric fields to said tissue.

Another objective of the present invention is to provide an electrode/ electrotherapeutic device geometry that is configured to minimize the trauma inflicted on intervening tissue during insertion and, if applicable, continued presence, of the electrodes.

Another objective of the present invention is to provide an electrode/ electrotherapeutic device that is configured to enable the precise application of an electric field to a specific tissue area without affecting surrounding or intervening tissue.

Another objective of the present invention is to provide an electrode/ electrotherapeutic device that is configured to enable the precise and predictable generation of complex, potentially irregular three-dimensional field shapes.

Another objective of the present invention is to provide an electrode/ electrotherapeutic device that increase the efficiency of transfer of electric fields, preferably without increasing the overall dimensions of the electrode/electrotherapeutic device.

Another objective of the present invention is to provide an electrode/ electrotherapeutic device that is configured to ensure optimal transfer of electric field into tissue.
It is a further object of the present invention to provide an alternative to the prior art devices.

It is a further object of the present invention to provide an improved electroporation, where an improved, controllable electric field may be obtained.

It is a further object of the present invention to provide an improved electroporation, where burning or scarring of tissue in or adjacent to the target tissue may be avoided or limited.

In particular, it may be seen as an object of the present invention to provide an electroporation device that solves the above mentioned problems of the prior art.

In the following, reference is made to an electrotherapeutic device. Hereinafter this term in this context is to be understood as an elongate member carrying one or more electrodes, where an electrode is to be understood as a conductive surface arranged for transmitting an electrical current.

### Summary of the invention

These and other objectives of the invention are obtained in a first aspect of the invention by an electroporation device for inducing an electrical field in the body of a patient comprising a plurality of electrotherapeutic devices having respective distal ends, each electrotherapeutic device being slidably arranged with respect to a reference point P on the electroporation device, from a retracted position, to an extended position, where said distal end extends distally beyond the position of said distal end when in said retracted position, wherein at least one, a group of or each of said electrotherapeutic device comprises
- an elongate main body part/wire section having a longitudinal axis, a distal end and a proximal end;
- an electrically conductive terminal tip disposed at said distal end of said elongate body part; and
- a first electrically conducting path extending along said elongate main body part from said proximal end to said distal end,
wherein said elongate main body part is of a substantially uniform cross sectional area, and wherein a maximal cross sectional area of said terminal tip in a direction perpendicular to said longitudinal axis is greater than the cross sectional area of said elongate main body part.

The objectives of the invention are further obtained by a second aspect of the invention by an electroporation device comprising an elongate introducer shaft, said introducer shaft having a distal tip; said plurality of electrotherapeutic devices being slidably arranged within said introducer shaft from a retracted position, where said terminal tips are enclosed within said introducer shaft or fully or partially within said distal tip or located at an outer surface of said distal tip, to an extended position, where said terminal tips extend from said distal tip to a plurality of laterally extending angularly spaced positions, and where at least one , a group of or each of said electrotherapeutic device comprises
- an elongate main body part/wire section having a longitudinal axis, a distal end and a proximal end;
- an electrically conductive terminal tip disposed at said distal end of said elongate body part; and
- a first electrically conducting path extending along said elongate main body part from said proximal end to said distal end,
wherein said elongate main body part is of a substantially uniform cross sectional area, and wherein a maximal cross sectional area of said terminal tip in a direction perpendicular to said longitudinal axis is greater than the cross sectional area of said elongate main body part.

By these aspects of the invention, a precisely definable and exactly controllable electric field may be generated. Further, the electric field may be tailored to specific, user-defined geometric configurations. Yet further, control of parameters of the electric field, i.e. shape and distribution of gradients as well as uniformity of the field, will be enhanced. Yet further, generation of fields with complex, three-dimensional geometries, i.e. field geometries based on placement of terminal ends of multiple electrotherapeutical devices in two or more separate planes will be enhanced. The precision of the specific, user-defined geometric configurations as well as the uniformity and homogeneity of said electric fields is enhanced by the specific shapes of said terminal tips.

Thus, the present invention aims at introducing a multitude of electrotherapeutical devices to surround the target tissue and to expose said tissue to brief electric pulses with the aim of permeabilization of cells. Therapeutic molecules can be brought into the treatment area with the aim of a) changing the electrical properties and b) as solvent for therapeutic molecules aimed for internalization in the permeabilized cells, or as adjuncts to the procedure (membrane resealing or enhancement of biological effect). The essentially spherical shape of the terminal tips of the electrotherapeutical devices will lead to a) better field distribution due to the enlargement of the transmitting surface from which the electric field originates, b) fewer hot spots due to the lack of sharp points, edges and corners, c) widest possible transmission angles (i.e. fields of view), enabling the electrotherapeutical device to transmit fields that are essentially unaffected by the direction of transmission, and enabling a given electrotherapeutical device to establish a field with substantially unchanged parameters with any other electrotherapeutical device within the field of view of that electrotherapeutical device

The electrode tip will be less likely to traumatise tissue by 'cutting', both when extended and withdrawn.

By the electroporation device according to the second aspect is further obtained that a plurality of electroporation devices may be brought into deeper lying regions of tissue in a minimally invasive way, by a single passageway, thereby eliminating unnecessary damage to e.g. healthy tissue.

According to this second aspect, the terminal tips of the electrotherapeutic devices may be deflectable away from a longitudinal axis of said shaft when deployed/extended to their extended position, such that at least one planar projection taken in a plane perpendicular to said longitudinal axis of a distance between a pair of terminal tips of said electrotherapeutic devices is larger than a maximal extent of a cross-section of said introducer shaft, said cross-section taken in a plane perpendicular to said longitudinal axis at a distal end of said introducer shaft.

The deflection of said terminal tips of said electrotherapeutic devices when in their extended position, may be provided by a curving of a plurality of distributor channels provided in at least said distal tip of the introducer shaft. Thereby a simple and efficient spatial distribution of the terminal ends may be obtained.

Alternatively or additionally the deflection of said terminal tips of said electrotherapeutic devices, when in their extended position, may be provided by a biasing of at least a section of said main body part of said electrotherapeutic devices.

The distal tip of the introducer shaft may preferably be formed with a substantially smooth, rounded, non-cutting shape with a substantially smooth, non-cutting transition to the introducer shaft. Thereby the introducer shaft may be introduced causing a minimum of trauma to the tissue

The distal tip may be detachable from said introducer shaft. Thereby a modular device using one time pre-sterilized parts may apply especially for those parts that are intended for contact with patient tissue may be obtained. Alternatively the tip is formed integrally with the shaft.

Each of said electrotherapeutic devices can be extended individually or in sets. Thereby the electrotherapeutic devices may be advanced all the way to their maximally extended position as defined by their lengths and/or by stops arranged in connection with the means for advancing/retracting the electrotherapeutic devices or they may be partially extended to a position extended from their retracted position. Thus a tailor-made spatial distribution of terminal tips may be obtained. Thereby, the extended distribution of the electrodes, and thus the shape of the electrical field, may be adapted to the individual target tissue. Alternatively, the electrodes may be advanced or extended from the tip in subsets of electrodes or as one set of electrodes, e.g. such that the length of the individual electrodes are adapted to the target tissue shape.

Said electrotherapeutic devices may be extendable such that their terminal tips form a spatial distribution around a volume of target tissue.

In an embodiment the device may be constructed such that the electrotherapeutic devices are extendable such that their terminal tips form a substantially spatial spherical or ellipsoid distribution pattern, having a circular or elliptical cross section taken in a plane parallel to said longitudinal axis when extended.

In an embodiment said electrotherapeutic devices are slideably arranged in electrically insulated guide channels formed in the introducer shaft and/or distal tip of the introducer shaft.

In an embodiment the introducer shaft further comprises a fluid delivery channel through which a dose of therapeutic molecules can be administered, said fluid delivery channel extending through the length of said introducer shaft and terminating through said distal tip, said fluid delivery channel being separate from said distributor channels.

In any of the above the electroporation device may comprise a handle section, said elongate introducer shaft extending from said handle section, wherein the handle section comprises an energy source for applying through said electrotherapeutic devices an electrical field to a target tissue, when the electrotherapeutic devices are in their extended position.

In an embodiment the handle section may comprise a therapeutic molecule delivery system comprising a therapeutic molecule reservoir and actuating means for administering said therapeutic molecules through said fluid delivery channel.

The handle section may further comprise a control unit to control the transmission of pulses from the device and/or the administration of the therapeutic molecules.

In any of the above embodiments said introducer shaft may have has a circular cross section with an outer diameter of 10 mm or less, preferably of 5 mm or less, more preferably of 3.5 mm or less.

In any of the above embodiments the introducer shaft may comprise an outer tube and an inner electrotherapeutic device assembly guide received in said outer tube, and where said electrotherapeutic devices are slideably arranged in guide channels formed in said inner electrotherapeutic device assembly guide.

In a further embodiment said guide channels are formed in a set of cylindrical guide sheaths that are received in longitudinal semi-open channels distributed radially along the periphery of said inner electrotherapeutic device assembly guide.

In any of the above embodiments each electrotherapeutic device may be assigned an individual electric polarity, such that the electric stimuli can be provided from and between individual electrotherapeutic devices. Thereby it is achieved that couples of electrotherapeutic devices may communicate electrically with each other in order to provide a clearly defined and configurable electric field in the target tissue. Further, by individually assignability of the electrotherapeutic devices a user may obtain a programmable electrical field. By programming the electroporation device or a an external controller device the shape of an electrical field between a multitude of terminal tips may individually configured to the contours of a given lesion or target tissue. The most precise configuration of an electrical field may be obtained momentarily at all time during use. Thus is provided an electrotherapeutic device array to be used for different lesions contours through selective assignment of pulses in a given sequence.

In any of the above embodiments the terminal tips may be enclosed within the distal tip when in their retracted position.

For example at least one, a group of or each terminal tip may be hidden within an enlargement formed in the distal-most end of the distributor channels provided in said distal tip, when in their retracted position.

Alternatively, said terminal tips may be located at an outer surface of said distal tip of said introducer shaft when in the retracted position and where the distal tip is covered by a dissolvable layer, such that said terminal tips are enclosed in said dissolvable layer, when in retracted position, and such that said dissolvable layer forms a substantially smooth, rounded, non-cutting shape with a substantially smooth, non-cutting transition to the introducer shaft.

The dissolvable layer may be of a kind that will gradually dissolve by contact to internal tissue of a patient or is dissolved by application of a suitable energy from the electroporation device itself, or from an external source.

In all of the above embodiments the terminal tips of said electrotherapeutic devices preferably have a smooth, rounded outer surface geometry. For example the terminal tips may be are elliptical, or they may be substantially spherical. Further, a transitional surface from the elongate main body part to the terminal tip of at least one, a group of, or each electrotherapeutic device may be smooth and rounded.

In an embodiment at least one, a group of, or each of said elongate main body parts of the electrotherapeutic devices, have circular cross sectional shapes in a direction perpendicular to said longitudinal axis.

In an embodiment the terminal tip and said electrically conducting path are formed in different electrically conductive materials.

In all of the above embodiments the elongate main body part may constitute said first electrically conducting path.

In all of the above embodiments the elongate main body parts may further comprise an outermost non-conductive, electrically insulating layer.

In an embodiment thereof at least one of said electrotherapeutic devices has a portion of said elongate main body part that is electrically un-insulated to have a second electrically conductive surface.

In a further embodiment said second electrically conductive surface(s) includes a point with a distance from and in a direction perpendicular to said central longitudinal axis greater than the maximum distance from any point on the outer surface of said elongate body part and in a direction perpendicular to said central longitudinal axis.

In a further embodiment said second electrically conductive surface is electrically connected to said first electrically conducting path.

In a further embodiment said second electrically conductive surface is electrically connected to a second electrically conducting path extending along said elongate main body parts from said proximal end to said second electrically conductive surface (161), said second electrically conductive path being electrically insulated from said first electrically conductive path.

In a further embodiment said second electrically conductive surface circumscribes the elongate main body part.

In a further embodiment said second electrically conductive surface shows a substantially rectangular cross sectional shape in a cross section parallel to said longitudinal axis.

In another embodiment an outer contour of said second electrically conductive surface defines a convex arc in a cross section parallel to said longitudinal axis.

The outer contour of said second electrically conductive surface may for example define a semi-circle in a cross section parallel to said longitudinal axis (B).

Preferably, a transition from the outer surface of said elongate main body part to the second electrically conductive surface is smooth and rounded.

The second electrically conducting path, extending along said elongate main body part, to said second electrically conductive surface may be a tubular structure being electrically insulated from said first electrically conducting path. The first electrically conducting path may thus be centrally structured within said main body part. Thus the first electrically conducting path may be a monofile wire.

In a further embodiment at least one main body part of an electrotherapeutic device has a fluid passage extending in the direction of said longitudinal axis through which a dose of therapeutic molecules can be administered from a reservoir connected to or connectable to the proximal end of said electrotherapeutic device. The fluid passage may be centrally located within said electrotherapeutic device, and where at least one of said first or second electrically conducting paths is a tubular conductor formed concentrically around said fluid passage.

In any of the above embodiments the main bodies of the electrotherapeutic devices may be of substantially circular cross-sectional form, and wherein the diameter of said main bodies is less than 2 mm, preferably less than 1 mm, more preferably less than 0.5 mm, and even more preferably less than 0.20 mm.

In any of the above embodiments the terminal tips of the electrotherapeutic devices has a maximum cross-sectional extent in a direction perpendicular to said axis of less than 3 mm, preferably less than 1,5 mm, more preferably less than 0.75 mm, and even more preferably less than 0.30 mm.

In any of the above embodiments having an outermost non-conductive, electrically insulating layer, said outermost non-conductive, electrically insulating layer may have a width of less than 1 mm, preferably less than 0.5 mm, more preferably less than 0.25 mm, and even more preferably less than 0.10 mm, and even more preferably substantially 0.025 mm.

In any of the above embodiments at least one, a group of, or each distributor channel may comprise a linear section provided distally to a curved section such that the path of an electrotherapeutic device deployed to its extended position is substantially linear.

In any of the above embodiments at least one, a group of, or each electrotherapeutic device (60) may be extendable to a position extending beyond the distal-most end of the distal tip of the introducer shaft.

In any of the above embodiments at least one, a group of, or each terminal tip of the electrotherapeutic devices may be extendable to a position extending beyond the distal-most end of the distal tip of the introducer shaft.

The objectives are further obtained by an electroporation method for creating one or more electrical fields to generate an electroporation and/or electrophoretic effect in a target tissue in the body of a patient, comprising the steps of
- providing an electroporation device comprising
   ∘ an elongate introducer shaft, said introducer shaft having a distal tip; and
   ∘ a plurality of electrotherapeutic devices having respective distal ends, wherein at least one, a group of, or each of said insertable electrotherapeutic devices comprises
      ▪ an elongate main body part/wire section having a centrally located longitudinal axis, a distal end and a proximal end;
      ▪ an electrically conductive terminal tip disposed at said distal end of said elongate body part; and
      ▪ a first electrically conducting path extending along said elongate main body part from said proximal end to said distal end,
      ▪ wherein said elongate main body part is of substantially uniform cross sectional area, and wherein a maximal cross sectional area of said terminal tip in a direction perpendicular to said longitudinal axis is greater than the cross sectional area of said elongate main body part;
   each electrotherapeutic device being slidably arranged within said introducer shaft from a retracted position, where said terminal tips are enclosed within said introducer shaft or distal tip or located adjacent to said distal tip, to an extended position, where said terminal tips extend from said distal tip to a plurality of laterally extending angularly spaced positions,
- inserting said introducer shaft through tissues of a body and bring said distal tip into a vicinity of a target region to be treated, while said electrotherapeutic devices are in said retracted position;
- extending said electrotherapeutic devices to said extended position to at least partially surround tissue in a target region of the patient; where the terminal tips create a virtual, three-dimensional enclosure of finite points to partially or fully enclose said target tissue; and
- Transmitting from one or more electrotherapeutical devices to one or more different electrotherapeutical devices one or more electric pulses of specific amplitudes and durations to create one or more electric fields in said target tissue.

The objectives are further obtained by an electroporation method for creating one or more electrical fields to generate an electroporation and/or electrophoretic effect in a target tissue in the body of a patient, comprising the steps of
• providing an electroporation device comprising a plurality of electrotherapeutic devices having respective distal ends, each electrotherapeutic device being slidably arranged with respect to a reference point from a retracted position, to an extended position, where said distal end extends distally beyond the position of said distal end when in said retracted position, wherein at least one, a group of, or each of said electrotherapeutic devices comprises
   o an elongate main body part/wire section having a longitudinal axis, a distal end and a proximal end;
   ∘ an electrically conductive terminal tip disposed at said distal end of said elongate body part; and
   ∘ a first electrically conducting path extending along said elongate main body part from said proximal end to said distal end,
   wherein said elongate main body part is of a substantially uniform cross sectional area, and wherein a maximal cross sectional area of said terminal tip in a direction perpendicular to said longitudinal axis is greater than the cross sectional area of said elongate main body part;
• inserting the terminal tips into the body of a patient at a desired location, and extending said electrotherapeutic devices to said extended position to at least partially surround tissue in a target region of the patient; where the terminal tips create a virtual, three-dimensional enclosure of finite points to partially or fully enclose said target tissue; and
• Transmitting from one or more electrotherapeutical devices to one or more different electrotherapeutical devices one or more electric pulses of specific amplitudes and durations to create one or more electric fields in said target tissue.

An aspect of the invention is to provide an electroporation device with a plurality of electrotherapeutic devices that comprises a spherically or ellipsoidically shaped distal terminal tip 61 suitable for percutaneous and intravascular applications.

Another aspect of the invention is an insertion mechanism in the form of an electroporation device comprising a single insertion passageway such as a needle, a laparoscope, a catheter or an endoscope comprising a plurality of electrotherapeutic devices that may be operatively connected to a pulse generator placed outside the body of a patient and may be used to generate an electric field of suitable amplitude and duration in a suitable location of the patient's body.
Yet another aspect of the invention is one of the above structures comprising a surface coating, e.g. a gene, chemotherapeutic agent or similar, that is released following application of e.g. electrical pulses.
Yet another aspect of the invention relates to an electroporation method inserting a set of electrotherapeutic devices of an electroporation device into the vicinity of a target tissue, applying a dose of therapeutic molecules and providing an electroporation by applying through said electrodes a sequence of electrical pulses to create a transient field in regions of the target tissue between alternating poles of the electrotherapeutic devices.

Yet another aspect of the invention relates to an electroporation method inserting through a singular passageway of an electroporation device a set of electrotherapeutic devices into the vicinity of a target tissue, applying a dose of therapeutic molecules and providing an electroporation by applying through said electrodes a sequence of electrical pulses to create a transient field in regions of the target tissue between alternating poles of the electrotherapeutic devices.

These and other aspects of the invention will be apparent from and elucidated with reference to the embodiments described hereinafter The aspects and embodiments described above and below are exemplary only.

### Description of the drawings

In the following the invention will be described in further detail with reference to the accompanying figures. The figures show ways of implementing the present invention and are not to be construed as being limiting to other possible embodiments falling within the scope of the attached claim set.
- Fig. 1 shows a diagram of the field strength around a set of prior art pointed electrodes/electrotherapeutic devices;
- Fig. 2 shows diagram of the field strength around a set of electrodes/electrotherapeutic devices in an electroporation device according to the invention;
- Fig. 3, in a principle outline, shows a section through an electrode/electrotherapeutic device constitution a part of an electroporation device according to the invention;
- Fig. 4 shows an embodiment of the electrode/electrotherapeutic device in Fig. 3, comprising a smooth transitional surface from a terminal tip to a main body part;
- Fig. 5 shows another embodiment of the electrode/electrotherapeutic device shown in Fig. 3, where a terminal tip is formed on a main body part;
- Fig. 6 shows another embodiment of the electrode/electrotherapeutic device in Fig. 3, comprising an electrically insulating coating on a main body part;
- Fig. 7, in a principle outline, shows a section through an electroporation device according to an embodiment of the invention having a set of electrodes/electrotherapeutic devices as shown in the previous figures;
- Fig. 8 shows another embodiment of an electroporation device according to the invention;
- Fig. 9, in a partly cut-out perspective view shows another version of an electrode/ electrotherapeutic device for an embodiment of the electroporation device according to the invention;
- Fig. 10, in a perspective view, in outline shows an embodiment an electroporation device according to an embodiment of the invention, having a plurality of electrodes/ electrotherapeutic devices as shown in Fig. 9;
- Fig. 11 shows a perspective view of an electroporation device according to an embodiment of the invention;
- Fig. 12 shows, in a perspective view, a distal end of a lateral cut through of an introducer shaft of an electroporation device according to an embodiment of the invention;
- Fig. 13 shows a section through a distal end of the device shown in Fig. 12, the electrodes/electrotherapeutic devices being in a retracted position;
- Fig. 14 shows a section through a distal end of the introducer device shown in Fig. 12, the electrodes being in an advanced position;
- Fig. 15 shows a perspective view of a distal end of the introducer shaft shown in Fig. 12, with an indication of the range of the advanced electrodes;
- Fig. 16 shows a partly cut-out, perspective view of an electroporation device according to another embodiment of the invention;
- Fig. 17 shows, in an exploded, sectioned perspective view, parts of a distal end of an introducer shaft of a the electroporation device shown in Fig. 16;
- Fig. 18, in an exploded view, shows details of the electroporation device shown in Fig. 16;
- Fig. 19, in a perspective view from the front (distal end), shows a distal tip of a device according to one embodiment of the invention, with two layers of extended electrode/electrotherapeutic device distal-most terminal tips visible in an extended position;
- Fig. 20 shows some of the electrodes/electrotherapeutic devices extending from the distal tip of the device shown in Fig. 19, indicating a pulse emitting pattern between these electrodes electrotherapeutic devices; and
- Fig. 21 shows the resulting pattern of the electric field induced in a target tissue by the pulse emitting pattern indicated in Fig. 20.

### Embodiments of the invention

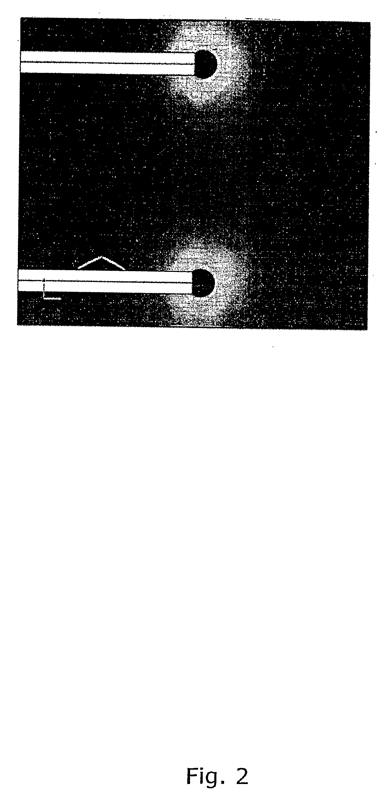

Figs. 1 and 2 shows the resultant electrical field strength computer simulation around two different sets of electrotherapeutic devices with point shaped electrodes (only the tips are un-insulated) of an electrical pulse between poles of the electrotherapeutic devices, the test being performed by the inventors. Fig. 1 shows the electrical field induced by a set of prior art electrotherapeutic devices having pointed ends. Around the edges and points provided by the angles between the surfaces of the electrodes, so-called hot spots of high intensive energy are created, which will create a burning or scarring of the tissue in the region of the hot spot. In ablation procedures this is not so relevant because the purpose is to cause cell death. However in electroporation this may be highly undesirable, since cell death in itself may be undesirable, or because it may change the electric properties of the tissue, making it difficult to control the process of applying the field. In Fig. 2 is shown how the formation of hot spots is substantially avoided by applying a similar pulse under the same conditions between a set of electrotherapeutic devices having rounded smooth and essentially spherical terminal tips.

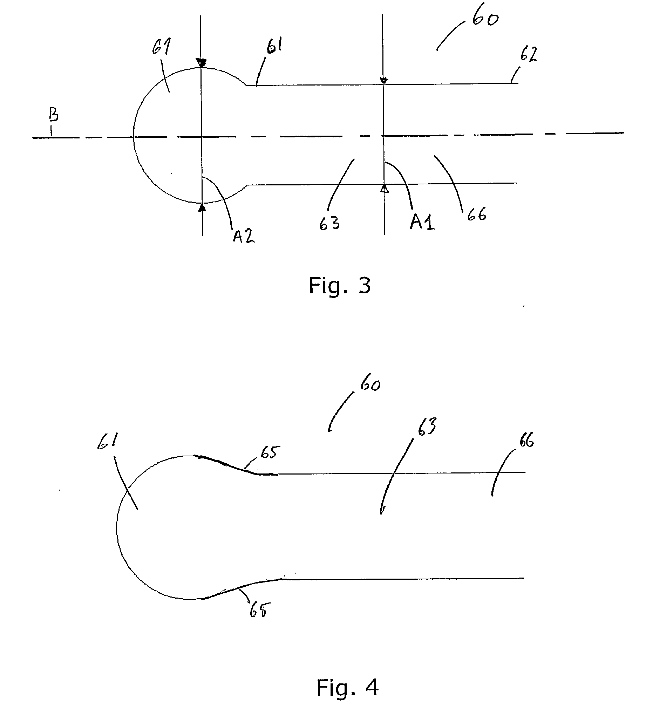

In Fig 3 is shown an electrotherapeutic device 60 for an electroporation device 1 according to an embodiment of the invention. The electrotherapeutic device 60 comprises an elongate main body part 63, e.g. in the form of an elongate wire section, preferably of uniform diameter, and a terminal tip 61 formed in a distal en 64 of the elongate main body part 63. The main body part 63 has a longitudinal axis B, a distal end 64 and a proximal end 62; an electrically conductive terminal tip 61 disposed at said distal end 64 of the elongate body part 63 a first electrically conducting path 66 extending along the elongate main body part 63 from said proximal end 62 to said distal end 64. In the embodiment shown the electrically conducting path 66 is constituted by the main body part 63. However, in other embodiments the electrically conducting path 66 may be formed on or in the main body part 63.

In the figured embodiment the terminal tip 61 of the electrotherapeutic device 60 is of circular cross sectional shape with a maximum diameter that is larger than a diameter of the cylindrical main body part 63 wire said sections taken in a plane perpendicular to the axis B of the main body part 10.

More generally an electrotherapeutic device 60 for an electroporation device 1 according to the invention may have an elongate main body part/wire section 63 of other cross sectional shapes than circular, e.g. oval. In the general case the elongate main body part 63 is of a substantially uniform cross sectional area (A1), and a maximal cross sectional area A2 of the terminal tip 61 in a direction perpendicular to said longitudinal axis B is greater than the cross sectional area A1 of the elongate main body part 63.

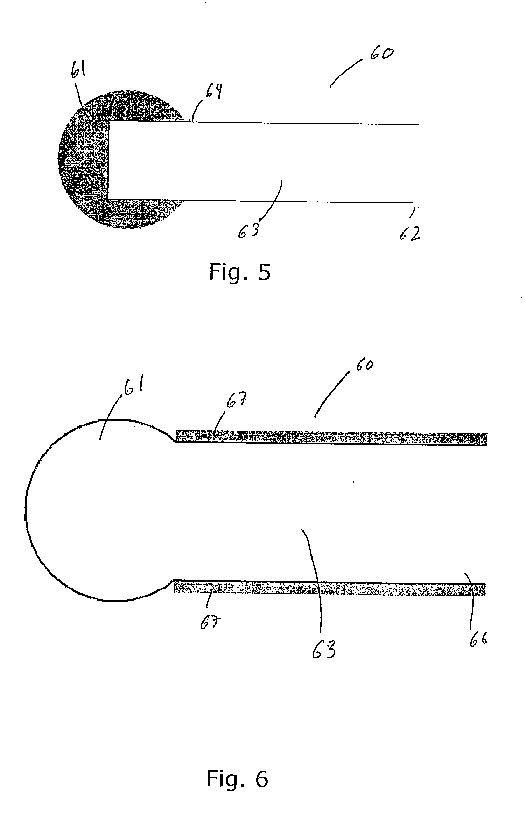

The main body part 63 and the distal tip may be of the same material or of two or more different materials, as long as both materials are highly conductive, see Fig. 5.

The terminal tip 61 may in a preferred embodiment be spherical. A spherical shape is particularly advantageous since an electric field that is applied through one such terminal tip 61 that is in electrical communication with one or more other terminal tips of an electroporation device 1 will be less distorted by boundary effects than what is accomplishable with other geometries (simulations Figs. 1-2). Therefore, the field applied from an electroporation device 1 having such electrotherapeutic devices 60 will be more homogeneous than with pointed or cutting electrotherapeutic devices 60.

A method of manufacturing such an electrotherapeutic device 60 comprises creating a spherical terminal tip 61 on a wire by heating said wire with a focused laser beam. The wire is preferably monofile, but multi-strand wires may also be used. The wire may for instance be made of a titanium alloy such as Ti - 6Al - 4V ELI, an alloy comprising platinum and iridium, such as Pt20Ir or a similar conductive material that exhibits desired characteristics such as biocompatibility, high stiffness and high conductivity. The laser used for the heating may for instance be an ND:Yag laser that is set at 210 V and delivers a laser pulse for 1.2 ms. Argon gas may be used as a shield gas to avoid oxidation of the material during welding. Alternative means of creating a spherical terminal tip 61 directly from the wire may be to use electron beam welding, arc-welding, or any similar means that provide sufficient control over tip geometry.

Alternatively, the spherical terminal tip 61 may be made as a separate component of the same material as the elongate wire section, or it may be made of an entirely different material, perhaps through moulding, turning, grinding or laser forming. Both of these embodiments may be appreciated from Fig. 5. Suitable means of joining the spherical terminal tip to the wire may be conductive glue, solder, laser etc. Similar considerations may apply for terminal tips 61 of other geometrical shapes such as e.g. elliptical.

Preferably the elongate wire section/main body part 63 of the electrotherapeutic device 60 is coated with a non-conductive, electrically insulating material, whereas the distal tip is conductive and non-insulated, see Fig. 6. In the figure the main body part is covered by a coating (67) that terminates in a distally facing edge immediately behind the terminal tip (61). An alternative embodiment comprising a coating 67 has a covering/coating with a distal end section that is flush with a proximal curvature, i.e. a transitional surface (65) of the terminal tip 61. Another preferred embodiment has a distal end section of the layer/coating 67 that slopes down to a point immediately behind terminal tip 61. This embodiment is advantageous as it may reduce/eliminate hot-spot effects if a non-smooth transition between wire section/main body and terminal tip is chosen, while at the same time providing substantially unrestricted transmission of a field from the widest possible transmission angle. Suggested (acute) angles of the sloping section of the coating/insulating layer may be 5, 10, 20, 25, 30, 35, 40, 45 degrees.

Coating/insulation of the wire section/main body is advantageous as it ensures that only the terminal tip of an electrotherapeutic device is electrically active. Thus, an electrically conductive terminal tip 61 may be clearly defined that allows superior control of the field that is applied through electrotherapeutic device 60. As described in further detail below, two or more electrotherapeutic devices 60 may be placed in a same tissue region, and an electric field may be created precisely between the conductive, non-insulated terminal tips, whereas the main body parts 63 of the two or more electrotherapeutic devices 60 will remain electrically inert. Thus, it is possible to precisely control field distribution, and fields may be given two-or three-dimensional shapes through the application of electrical pulses from multiple electrotherapeutic devices 60.

One exemplary production method whereby the wire sections/main bodies of electrotherapeutic devices 60 may be rendered electrically inert comprises the following:
• coating the elongate main body part 63 and the terminal distal tip 61 with a suitable non-conductive, electrically insulating material. The non-conductive, electrically insulating material may for instance be a fluor-ethylene-propylene (FEP), Parylene, or a similar material that exhibits desired characteristics such as biocompatibility, chemical inertness, high dielectric strength, resistance to abrasion and low friction. Coating may be applied by means of spray, vaporization, dipping or similar means, and curing may be by means of drying, chemical reactions, UV radiation or similar means. Exemplary widths of coating are below 2 mm, preferably below 0.5 mm.
• Following the coating of the entire electrotherapeutic device 60 the non-conductive, electrically insulating material is removed from the distal terminal tip 61 by directing a low-strength focused laser beam at distal tip, thereby burning away material without affecting wire section/main body part 63 or terminal tip 61. The laser used for the removal may for instance be a CO2 laser that is set at a frequency of 500 Hz and a power level of 30W.

An alternative method may include using a mask to cover the terminal tip of the electrotherapeutic device during coating, thereby eliminating the process step of removing the coating from the terminal tip with a laser.

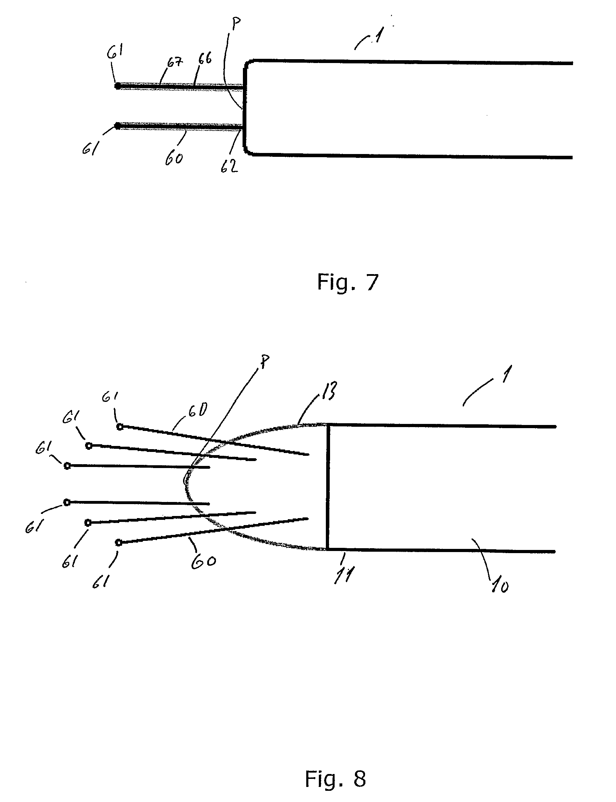

As shown in Fig. 7, and as described in further detail below, the application of the electrotherapeutic devices 60 described above would comprise inserting two or more electrotherapeutic devices 60 through an inserter configured as an endoscope, a catheter or a laparoscope. These two or more electrotherapeutic devices 60 may then be brought to communicate electrically by suitable manipulation of the polarities of the terminal tips 61. Such selective communication may for instance be used to create two-or three-dimensional electric fields in human tissues as part of a treatment.

One particular embodiment of this electroporation device 1 includes providing a distal tip 13 that is configured to impose on the electrode leads certain paths. By suitably configuring said distal section, the electrotherapeutic devices 60 may be deployed in a way that permits terminal tips 61 to define specific geometric structures through their spatial relations.

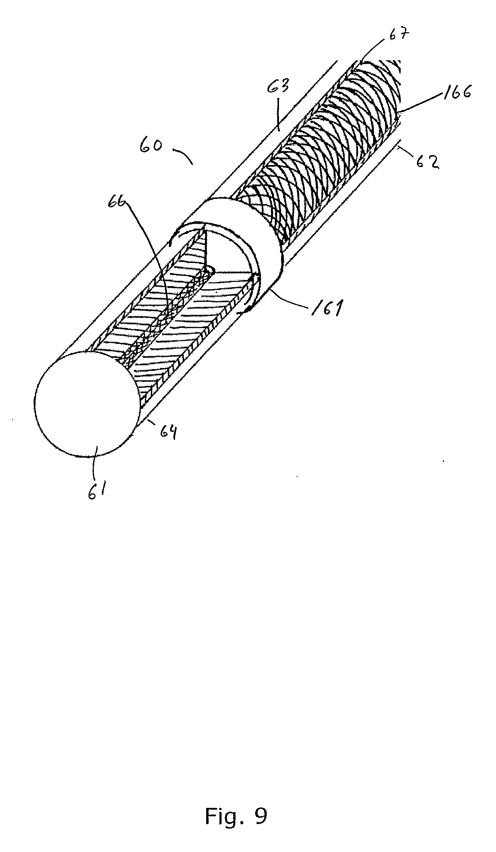

In some embodiments of the electroporation device 1, and with reference to Fig 9, one or more multipolar electrotherapeutic devices 60 may be used either in combination with electrotherapeutic device 60 as described above or solely a plurality of multipolar devices. The multipolar electrotherapeutic device 60 comprises an elongate a main body part 63, preferably of uniform diameter, and a conductive, spherical terminal tip 61 as described above. Proximally to the distal terminal tip 61, and coaxially disposed about said elongate main body part 63, is placed one or more annular conductive surfaces 161 that may for instance be flat. Annular conductive surfaces 161 of other shapes, such as semi-circular, are also envisioned. Said annular conductive surfaces have inner diameters that are larger than the outer diameter of the elongate main body part 63, and outer diameters that may be approximately similar to that of the terminal tip 61. In some embodiments, the annular conductive surfaces 161 may be electrically insulated from the first electrical pat 66 and from each other, and may be electrically connected to one or more electrical leads that may be assigned polarities that may for instance be neutral or may be the opposite of that of the terminal tip 61.

In a particular embodiment, a duo-polar electrotherapeutic device 60 comprises an inner core formed by e.g. a conductive, monofile wire 66. Said wire 66 is electrically connected with the terminal tip 61 that may be of a similar material as the wire 66, alternatively of another material with similar electrical properties. The terminal tip 61 has a diameter that is larger than the diameter of the inner core Said inner core is coated with a layer of coating material with high dielectric strength, except for a distal portion that is joined with the terminal tip 61. Said inner core may be fixedly enclosed in the coating material, or it may be movable to e.g. impose a torque of a certain magnitude or direction on the terminal tip 61. Said coating material may be firmly or semi-firmly attached to the terminal tip, or it may have no attachment.

Proximally to the distal tip, a single annular conductive surface 161 is coaxially disposed about the coating material and attached to said material. Attachment may e.g. be through application of glue or by sinking the annular conductive surface 161 into the outer surface of the coating during hardening. A particular embodiment comprises a section of coating material that surrounds the inner core in the space between the terminal tip 61 and the conductive surface 161 and has a wall width that is marginally smaller than the outer diameters of the terminal tip 61 and the annular conductive surface 161 in a plane perpendicular to the axis of the inner core. Also disposed about the coating material, and electrically connected to the annular conductive surface 161, is a conductive lead 166 comprising a conductive sleeve 166 that may be made of e.g. braided metal wires or similar conductive materials. Said sleeve covers the coating material proximal to the annular conductive surface 161 and permits the independent assignment of a specific polarity to the annular conductive surface 161, while a different polarity may be assigned through the inner core to the terminal tip 61.
Enclosing and electrically insulating the conductive sleeve is an outer sheath that is made of a non-conductive material with high dielectric strength. The outer sheath covers the conductive sheath proximal to the annular electrode and may also cover the section between the annular conductive surface 161 and the terminal tip.

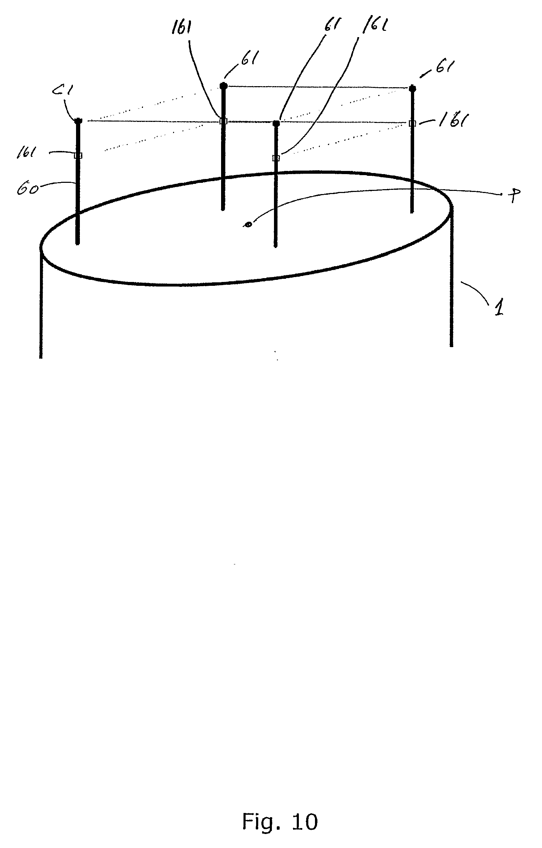

Such an electrotherapeutic device 60 configuration will allow the creation of an electric field along a single electrotherapeutic device 60 and provide a multitude of combination possibilities for creating electrical fields of varying geometrical shapes when applied through an electroporation device 1 having a plurality of such electrotherapeutic devices 60, as shown e.g. Fig. 10 and as further described below.

For instance, Fig. 10 shows four electrotherapeutic devices 60 comprising each a terminal tip 61 and a single annular conductive surface 161 that may be deployed along linear, parallel paths to form a box-shaped geometry.

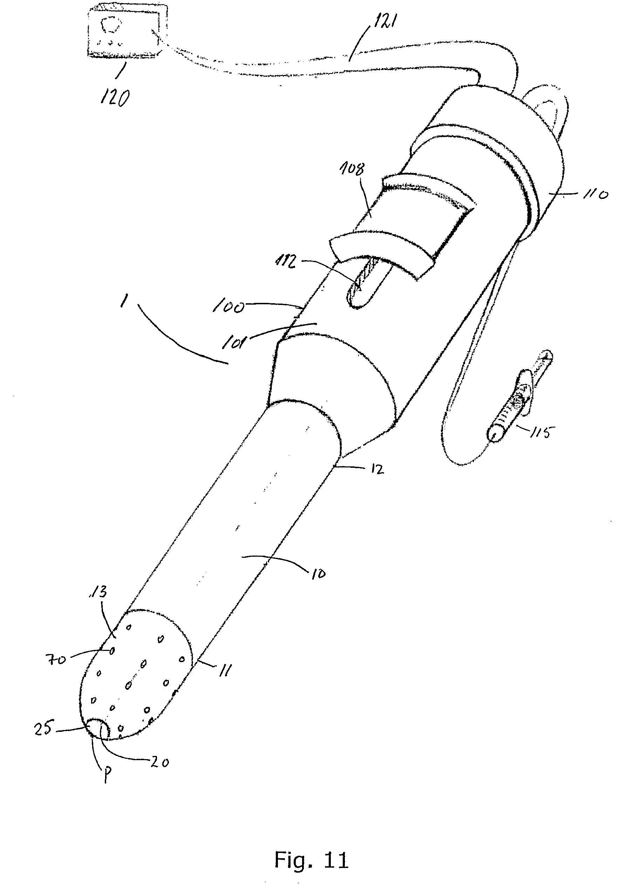

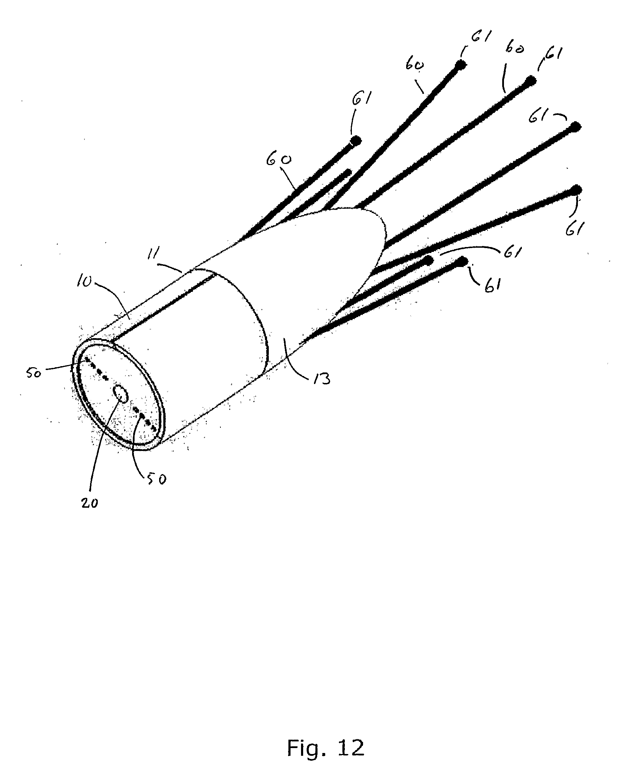

In Fig. 11 an electroporation device 1 according to an embodiment of the invention is shown, which device 1 is particularly suitable for introducing electrotherapeutic devices as described above into deeper lying tissues of a patient, and in particular into the brain. The device 1 comprises a handle section 100 and an elongate introducer shaft 10 preferably having a length suitable for accessing deeper-lying tissue regions. The length of the shaft 10 may be adapted for the intended use. The shaft 10 is attached to the handle section 100, and has a proximal end 12 adjacent to the handle section 100 and a distal end 11. The shaft 10 may in one embodiment be fixedly attached to the handle section 100. In other embodiments the shaft may be detachably mounted to the handle section 100, and may comprise suitable means for establishing temporary connections, e.g. for conducting electrical pulses to electrotherapeutic devices 60 arranged slidably within shaft 10 (See e.g. Figs.12-14). A distal tip 13 that is preferably shaped to permit the creation of a channel through intervening layers of tissue while causing minimal damage to said tissue when inserted into the body of a patient is disposed at the distal end 11 of said shaft 10. The distal tip 13 has a rounded, non-cutting shape. In other embodiments (not shown) the distal tip may be provided with a cutting edge or a pointed tip, i.e. a sharpened tip. These latter embodiments are e.g. well-suited for percutaneous applications. In either case, the distal tip 13 may be formed integrally with the introducer shaft 10 or it may be formed as a separate part coupled to the distal end 11 of the introducer shaft 10. With a removable/detachable tip 13, and/or a detachable shaft 10, the length and thereby the reach of the device, may be adapted to individual uses, by replacement with a suitable choice of shafts. Further, this allows for use of pre-sterilized single-use only parts for the parts that are inserted into a patient. Thereby, the need for disinfection of the parts to be inserted into a patient may be eliminated.

The introducer shaft 10 comprises a centrally located delivery channel 20 provided through the shaft 10 from the proximal end 12 to the distal end 11 along a longitudinal axis, L, of said shaft 10, and terminating through said distal tip 13, said channel 20 having a proximal end 22 and a distal end 21. At the distal end 21 of the channel 20 one or more outlets 25 are provided in the distal tip 13 in order to administer an amount of fluid/medical compound adjacent to the distal tip 13. In the embodiments shown in the figures a single outlet 25 is provided, however, the channel 20 may split up into a multitude of minute channels at the distal end 21, each having an outlet at the distal tip 13. The proximal end 22 of the channel 20 extends through the shaft 10 to the handle section 100, and is adapted for connection to a drug/genetic material delivery means 115 comprising a storage of a drug/medicament and/or means (e.g. a pump or a piston or the like) for advancing said medicament from said storage and through said channel 20 to a target tissue. In a simple form the delivery means may be provided by a syringe 115, connected to the delivery channel 20 via the handle section 100, e.g. by a tubing.

In an alternative embodiment (not shown), the channel 20 may be configured to receive an elongate delivery system, e.g. in the form of a tubing, that may reach from the storage means into the region to be treated. Such a delivery system may comprise a syringe connected to said tubing, in such a way that the channel is adapted to receive e.g. a distal section of said tubing.

In yet another alternative embodiment (not shown), the device 1 may provide an integrated therapeutic molecule delivery system comprising delivery means with advancing/pumping means and/or a storage for a medicament/drug, isotope or a genetic material solution, being integrated in the handle section 100.

The electroporation device 1 and the delivery channel 20 may also be configured by e.g. appropriate coupling means and/or dimensioning to receive and guide for instance an ultrasound probe, a surgical tool or another tool for minimally invasive manipulation of tissue. Thus the device 1 can be used in a flexible way, where for example it is not necessary to remove the device 1 and replace it with another specialized surgical tool, if the operator/surgeon encounters unexpected obstacles/difficulties prior to, during or following the electroporation process.

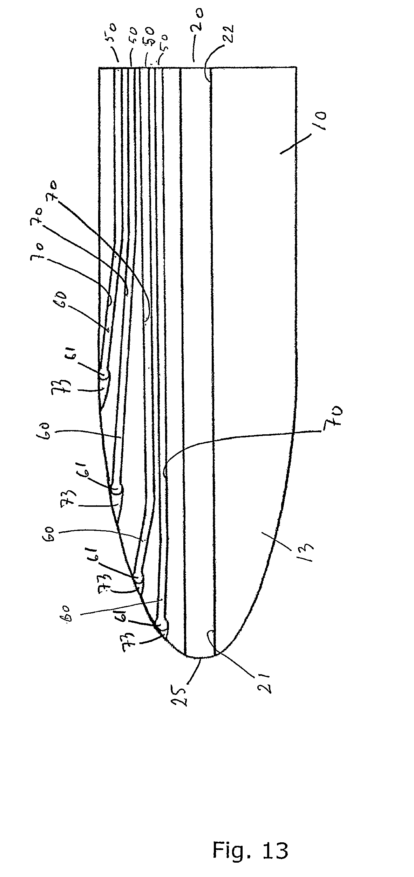

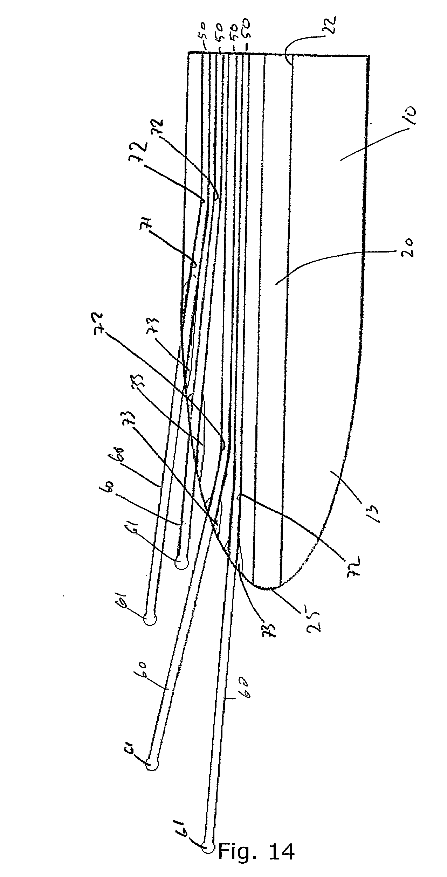

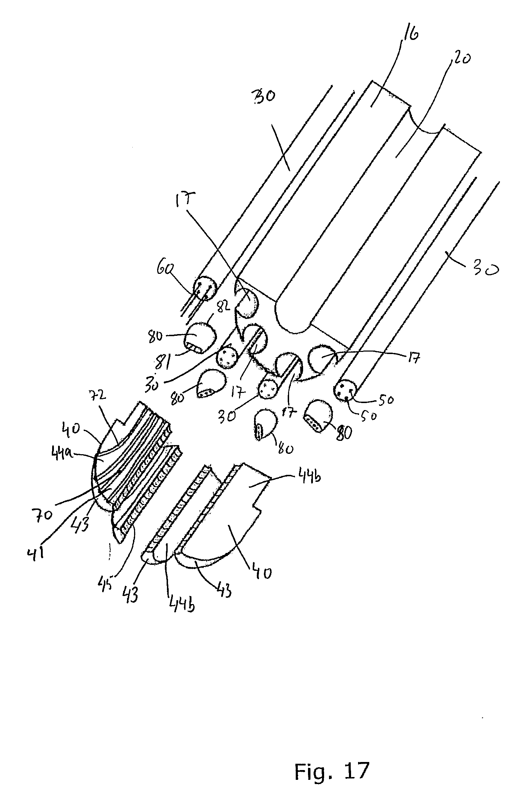

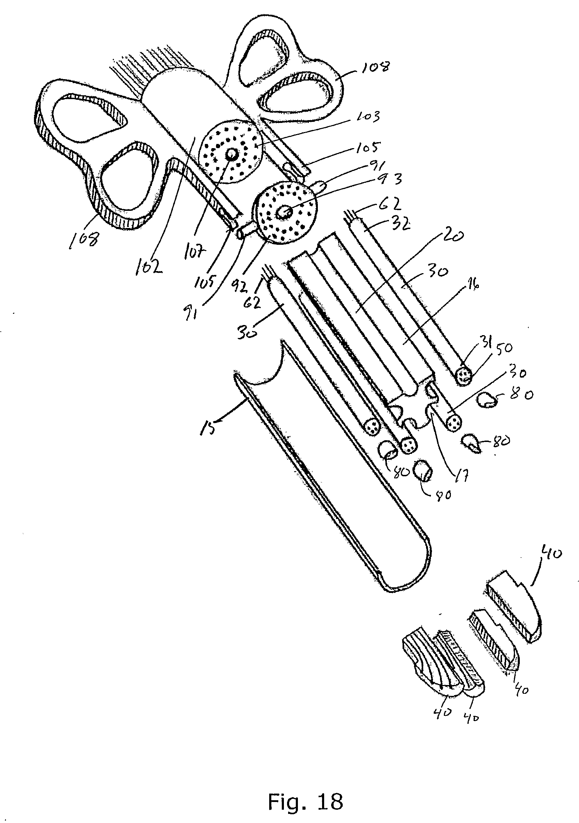

The shaft 10 further comprises a plurality of guide channels 50 (see Figs. 13-18), distributed around the central channel 20, and extending from the proximal end 12 to the distal end 11 of said shaft 10, and through the distal tip 13. Each guide channel 50 is adapted for guiding one or more elongate electrotherapeutic devices 60 that are movable relative to the shaft 10 between a first retracted position, as shown in Fig. 13, and a second extended position, as shown in Fig. 14.

In an alternative embodiment (not shown) each guide channel 50 may be provided, at least along a section of the shaft 10, by individual tubes, the shaft 10, in said section being formed by the set of individual tubes.

Each electrotherapeutic device 60 has a proximal end 62, extending into the handle section 100, a terminal tip 61 and an main body part 63 electrically connecting the proximal end 62 and the terminal tip 61 of each electrotherapeutic device 60.

The proximal ends 62 of the electrotherapeutic devices 60 are configured to act as connectors, thus providing a means of connecting the electrotherapeutic devices 60 to an electric stimulus generator 120 that supplies single electric pulses or sequences of electric pulses according to electroporation protocols for drug and gene delivery. The electric pulses are intended to generate an electric field for the purpose of creating transient permeabilization of cell membranes and/or an electrophoretic effect in the vicinity of the terminal tips 61 of said electrotherapeutic devices 60 when the introducer device 1 is placed in or close to a target tissue area and the electrotherapeutic devices 60 are forwarded to an extended position, see further regarding the use of the device below.

The electrotherapeutic devices 60 are connectable to an external electric stimulus generator 120 via an electronic connector (cable) 121 at the handle section 100 as shown in Fig. 10. In an alternative embodiment an electric stimulus generator 120 may be formed integrated with the introducer device, preferably in the handle section 100.

The configuration of the proximal ends 62 of the electrotherapeutic devices 60 further permits movement of the electrotherapeutic devices 60 between a first retracted position and a second extended position in a deployment sequence that will be further described below.

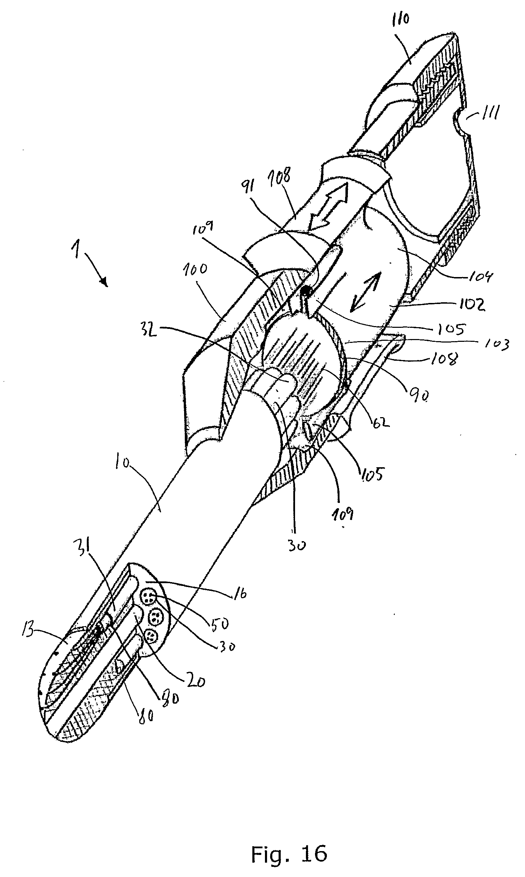

The main body parts 63 of the electrotherapeutic devices 60 are movably received in said guide channels 50 running through the introducer shaft from the proximal end 12 to the terminal tip 11 at the distal tip 13. Preferably, each electrotherapeutic device 60 has its own channel 50 to support and protect it and insulate it from the other electrotherapeutic devices 60, as shown in Figs 13-14, but multiple channels 50 may be bundled together in electrotherapeutic device assemblies, for example as shown in Figs. 16-18. Said guide channels 50 permit longitudinal movement of the electrotherapeutic devices 60 between the first retracted position and the second extended position.

Electrotherapeutic device 60 terminal tips 61 at the distal ends 64 of the electrotherapeutic devices 60 are movably received (as will be described in further detail below) in distributor channels 70 formed in the distal tip 13, which channel 70 are extending to the outer surface of said distal tip 13. Each distributor channel 70 further communicates with a corresponding guide channel 50 in the shaft proper 10. Thus, movement of the electrotherapeutic devices 60 in a longitudinal direction (with respect to the longitudinal axis of the shaft 10) between a first retracted position where the distally disposed end points 61 of the electrotherapeutic devices 60 are concealed within the distal tip 13, and a second extended position, where the end points 63 of the electrotherapeutic devices 60 are extended from the distal tip 13, is allowed.

In an alternative embodiment (not shown), the device may only have distributor channels 70 formed in the distal tip 13, the electrotherapeutic devices 60 being contained in a hollow shaft 10, the individual channels 50 being left out.

While positioned in the first retracted position, which is the default mode of the device 1, the end points at the terminal tips 61 of the electrotherapeutic devices 60 are held in storage in the distributor channels 70 in the distal tip 13, thus permitting the minimally invasive insertion of the device 1, i.e. with minimal damage to surrounding tissue.

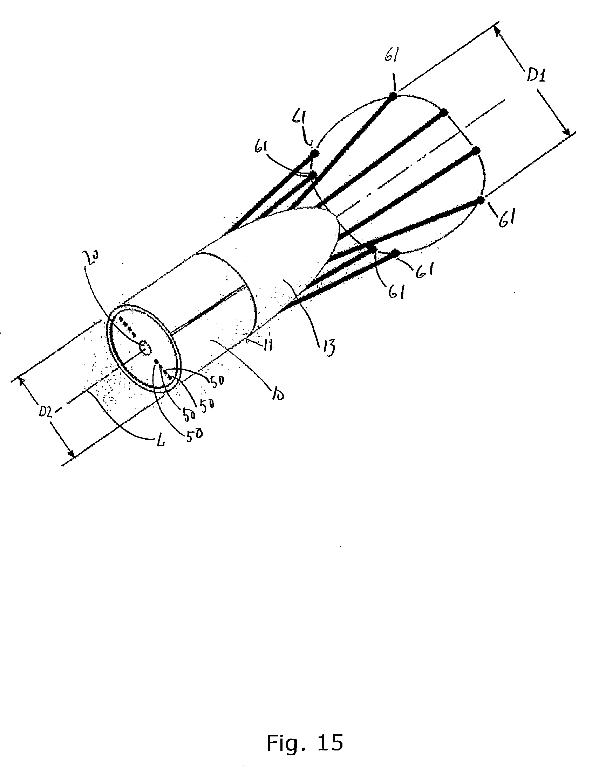

The distributor channels 70 are shaped to ensure deployment of the terminal tips 61 of the electrotherapeutic devices 60 in a predetermined pattern where a largest distance D1 (See Fig. 15) between a pair of oppositely arranged electrotherapeutic device end points 61, in a plane transversal to the longitudinal axis of the introducer shaft is larger than the diameter - or the largest extension D2 of the introducer shaft 10/distal tip 13 - in a plane perpendicularly to the longitudinal axis of the introducer shaft 10. Thus, it is made possible to access deeper lying tissues, e.g. within the brain, trough a single channel using a single introducer shaft 10, spreading the intervening tissues during the insertion, and, when the tip 13 reaches the target tissue, the electrotherapeutic devices can be extended through and/or around the target tissue. This allows an operator (surgeon) to treat a target tissue region or volume which has a cross-sectional dimension/extent larger than the diameter of a cross-section of the introducer shaft 10, where the cross-section is taken in a plane perpendicular to the longitudinal axis of the introducer shaft 10. In order to provide the above described distribution of the terminal tips 61 of the electrotherapeutic devices 60, the distributor channels 70 are formed such that at least some of the distributor channels 70 curve outwardly, i.e. away from a longitudinal centre axis L of the introducer shaft 10 (as seen from their connection to the distal end 11 of the corresponding guide channels 50 in the shaft 10 and towards the outer surface of the distal tip 13 where the distributor channels 70 terminates). Each of the distributor channels 70 or sets of distributor channels 70, may be provided with a different individual shape/deflection/curving 72 in order to ensure a specific pattern or distribution of the extended electrotherapeutic devices 60 during use. In a preferred embodiment, each distributor channel 70 has a linear or substantially linear section 71 distally of a curved section 72 in order to ensure a linear path through the tissue of the electrotherapeutic devices 60 when extended from their retracted positions.

Alternatively, the deflection away from said longitudinal axis L may be provided by e.g. a pre-tensioning or biasing of said electrotherapeutic devices 60. Such tensioning may be provided by a suitable choice of materials, e.g. a shape memory alloy such as Nitinol, or by forming the (flexible) electrotherapeutic device e.g. in a bent shape, such that when it is arranged in a straight guide channel 50 of the shaft 10 it is held in tension. The individual electrotherapeutic devices 60 or set of electrotherapeutic devices may have an individual biasing such that the electrotherapeutic devices may, when extended from their retracted position in the shaft 10/tip 13 form a desired spatial pattern around the target tissue.

Further, the desired spatial distribution of the part of the electrotherapeutic devices extending from the tip 13 may be provided by a combination of the shape of the tip distributor channels 70 and a biasing of the electrotherapeutic devices 60.

The bulging terminal tips 61 of the electrotherapeutic devices 60 may as shown in Figs. 13 and 14 advantageously be retractable into the distal tip 13, such that the terminal tips 61 are enclosed within the general outer surface defined by the tip 13 of the introducer shaft 10, by an enlargement 73 of the distributor channel 70 at the distal-most end thereof. Thereby, the distributor channel 70 (and the guide channel 50 are adapted to the cross-sectional width of the main body part 63 of the electro therapeutic device 60 and the enlargement 73 is adapted to the size of the bulging terminal tip 61. Alternatively, the entire distributor channel 70 or the channel 70 and the guide channel 50 may be adapted in cross sectional width to allow passage of the terminal tip 61.

In another embodiment the bulging terminal tips may in their most retracted position be located in a position directly on the outer surface of the distal tip 13. Thus the terminal tips would form bumps on the surface of the tip 13. In order to conceal the terminal tips and provide a smooth outer surface the tip 13 may be provided with a dissolvable coating enclosing the terminal tips. Such a dissolvable coating may e.g. comprise glucose, or another suitable material. The material may be dissolvable, e.g. over a suitable time (of a few seconds to a minute) when exposed to internal tissue of the body of a patient, or it may be dissolved by application of an energy applied e.g. through the electroporation device. Also the dissolvable coating may be dissolved by applying energy from outside the body of the patient.

Such a coating may also be provided to cover or smooth over gaps between the terminal tips 61 and the enlarged portion 73 of the distributor channel 70 at the outer surface of the tip 13.

In use, the electronic connection means (not shown) at the proximal ends 62 of the electrotherapeutic devices 60 are connected to a suitable electric stimulus generator 120. The shaft 10 of the introducer device 1 is then inserted, e.g. through a bore hole in a patient's skull or an incision in the patient's skin and introduced to the target region of the patient's body. The precise location of the target region and thereby for the bore/incision may be identified by means of ultrasound, CT, MR or another suitable means, and the correct position of the tip 13 of the introducer shaft 10 (post insertion) may be verified by similar means prior to, during or after deployment of the electrotherapeutic devices. When a correct position of the tip 13 of the introducer shaft 10 has been obtained relative to the target tissue, an operator may deliver a suitable chemotherapeutic agent, in fluid or liquid form, or a dose of genetic material or other substance through the delivery channel 20 and into the tissue region to be treated.

Before, during or after delivery of the drug or genetic material through the delivery channel, the operator may deploy some or all the elongate electrotherapeutic devices 60 in a desired pattern. Deployment is performed by actuating a suitable deployment mechanism at the handle section 100 or at the proximal end 12 of the shaft 10, and results in the longitudinal motion of all or some the electrotherapeutic devices 60 along the axis of the introducer shaft 10 from the first retracted position - as shown in Fig. 13 - to the second advanced position, e.g. as shown in Fig. 14. The distributor channels 70 in the distal tip 13 may be shaped to provide each individual electrotherapeutic device 60 with a unique path through the tissue, when advanced from the tip 13, which enables the creation of an electrotherapeutic device pattern where a distance D1 between oppositely arranged electrotherapeutic device end points 61 in a plane transversal to the longitudinal axis of the introducer shaft 10 is larger than a diameter D2 (or the largest extent of the shaft 10 in a section perpendicular to the longitudinal axis of the shaft 10 if the shaft is not of circular cross section) of the introducer shaft 10 in the same transversal plane.

Upon deployment of some or all of the electrotherapeutic devices to their extended position, an operator may actuate the electric stimulus generator 120 to deliver one or more pulses, e.g. a sequence of short and intense pulses to the tissue to be treated (target tissue). To ensure a suitable distribution of pulses and the thereby induced electric fields in the target tissue, pulses may be assigned to alternating specific electrotherapeutic devices 60 in a sequential pattern that may be tailored to suit the anatomy of the individual region of the body to be treated and/or the geometry of the specific malignant target tissue. Such assignment may be obtained for instance by suitable manipulation of the electric stimulus generator, e.g. through programmable electronic control means.

Upon pulse delivery, the operator may retract the elongate electrotherapeutic devices 60 to their retracted position by suitably manipulating the deployment mechanism in the handle section 100, and the device may be removed from the body of the patient. Alternatively, the operator may reposition the device 1 after having retracted the elongate electrotherapeutic devices 60, potentially permitting multiple pulse applications covering a larger area in a single device insertion.

The electroporation device 1 shown in Figs. 11-15 is depicted as having eight guide channels 50, distributor channels 70 and electrotherapeutic devices 60. However, a device according to the invention may be provided with any number of electrotherapeutic devices 60. The distribution of the guide channels 50 over a cross-section of the introducer shaft 10 shown in Figs. 11-15, is such that the electrotherapeutic devices all run in a plane parallel to the longitudinal axis of the introducer shaft 10. However, the electrotherapeutic devices 60 and their guide (and distributor) channels 50 (70) may be located around the entire circumference of the channel 20 in the shaft 10, surrounding the delivery channel 20 in other patterns as well.

Preferably, each of the electrotherapeutic devices are formed with an electrically insulating coating or sheathing, such that only the terminal tips 61 are conductive in order to form point electrodes. Thus, the electric pulses will create an electric field spanning the distance from point to point (terminal tip 61 to terminal 61), and a readily controllable firing pattern and thus a more controllable and accurate electric field may thus be generated by suitable selection and assignment of electrotherapeutic devices. For completeness it is to be understood that the entire length or part of the entire length of the electrotherapeutic devices 60 may also be electrically un-insulated, provided that the guide channels 50 and the distributor channels 70 are formed in an electrically insulating material.

As shown in Fig. 15 the device may be configured such that the terminal tips 61 of the electrotherapeutic devices 60 may form an ellipsoid field E that is the result of this electrotherapeutic device 60 pattern. A target tissue could be imagined situated within the ellipsoid area E, shown in the figure. Some of the electrotherapeutic devices 60 are thus advanced through the target tissue when guided to their extended position. In other embodiments of the invention it can be imagined that electrotherapeutic device patterns can be formed, such that a target area can be surrounded by electrotherapeutic device points (terminal tips) 61 in various three-dimensional patterns, e.g. a spherical or spherically elliptic or ellipsoid pattern.

As can be appreciated from Fig. 15, a device according to the invention may be adapted with sets of electrotherapeutic devices 60 that may be extendable to different distances from the distal tip 13 along the longitudinal axis of the shaft 10, such that the terminal tips 61 of each set are positioned in a common plane perpendicular to the longitudinal axis of the shaft 10. In Fig. 15 four sets of two electrotherapeutic devices extend to different distances from the distal tip 13, thus forming the above mentioned ellipsoid shape E.

Further, some of the electrotherapeutic devices 60 may be formed in such a way that they undertake a curved path through the tissue such that when advanced forward towards their extended position they will initially be deflected away from the central longitudinal axis L of the shaft 10, and will then reflect back such that the distal tip closes in on the central, longitudinal axis of the shaft 10, when advanced further. Thus, when fully extended, such an electrotherapeutic device 60 will describe a gently U-shaped or substantially, softened Ω-shaped curve. This may be accomplished by providing electrotherapeutic devices in an elastic material or a shape memory alloy such as Nitinol or by providing different section (lengthwise) of the electrotherapeutic devices with different biases (pre-tensionings).

Yet further, guiding channels may be shaped to impose on the electrotherapeutic devices certain paths through the tissue. For instance, it may be advantageous to impose on the electrotherapeutic devices a strictly linear path through the tissue, as the electrotherapeutic devices will then be able to withstand much higher loads without buckling - as opposed to electrotherapeutic devices given a curving path.

The deployment mechanism for the electrotherapeutic devices 60 may be manually driven or motorized (e.g. electronically controlled). The deployment mechanism may be adapted to advance all electrotherapeutic devices simultaneously as a set, or individually, or in groups (subsets) of electrotherapeutic devices 60. When the electrotherapeutic devices are advanced simultaneously, different electrotherapeutic device patterns may be achieved through a predetermined composition of electrotherapeutic devices of suitable lengths, shapes (by tensioning, alternative cross-sections predisposing the wire for certain directions of movement or by adequate shaping of guide channels) and materials. The device 1 according to the invention may further be controlled by an electronic control unit (not shown), either incorporated in the device 1 or connectable to the device 1 through a cable or a wireless connection. In the wireless configuration, a suitable power supply is preferably located inside the device. The electronic control unit may be programmable, such that a desired electrotherapeutic device pattern may be programmed prior to a surgical procedure.

In alternative embodiments (not shown), and as mentioned above, a partially disposable device variation of the above described embodiments is proposed, with a disposable introducer shaft 10 and non-disposable (re-usable) handle section 100 comprising a deployment mechanism with interfaces to electrotherapeutic devices formed in the disposable introducer shaft 10 and a electronic connections that may be customized to individual electrical stimulus generators 120.
The shaft may in all embodiments be formed in a plastic or metallic material such as titanium, stainless steel or an injection moulded polymeric material. The outer diameter of the shaft is preferably five (5) millimetres or smaller, preferably between gauge 17 to 14 incl. The wall width of the shaft is preferably between 0.05 mm and 0.25 mm. The guide channels 50, 70 may be formed in a suitable material, e.g. formed in a thermoplastic elastomer or a similar electrically insulating material. The electrotherapeutic devices 60 may be formed in an electrically conductive material such as titanium, stainless steel or the like In the following, an aspect of the invention, suited in particular for applications within the brain, e.g. in the treatment of brain cancer or genetic deficiencies will be described in further detail with reference to Figs. 16-18. Like references will be used for similar parts, with respect to the aspects of the invention shown in the previous drawings. The electroporation device 1 comprises an introducer shaft 10 and a handle section 100. The introducer shaft 10 is intended for insertion into the body of the patient and is fixedly attached to the handle section 100.

In alternative embodiments a partially disposable device is proposed, with a disposable introducer shaft 10 and non-disposable (re-usable) handle section 100 comprising a deployment mechanism with interfaces to electrotherapeutic devices formed in the disposable introducer shaft 10 and a connector that may be customized to individual electric stimulus generators 120.

The introducer shaft 10 shown in the figures comprises the following (in other embodiments some of the features may be omitted):
- An outer tube 15 having a proximal end 12 and a distal end 11 which is preferably formed in a plastic or metallic material such as titanium, stainless steel or an injection moulded polymeric material. The outer diameter D2 of this tube is preferably five (5) millimetres or smaller. The wall width of said outer tube is preferably between 0.05 mm and 0.25 mm and the length of the tube is preferably between 50 mm and 500 mm depending on the particular application.
- An inner electrotherapeutic device assembly guide 16 that is preferably formed in a thermoplastic elastomer or a similar electrically insulating material. The inner electrotherapeutic device assembly guide 16 is placed in an inner lumen of the outer tube 15. The electrotherapeutic device assembly guide 16 has a flattened proximal end and a flattened distal end comprising faces that lie perpendicular to the longitudinal axis. This electrotherapeutic device assembly guide 16 comprises eight straight, semi-open channels 17 distributed in a circular pattern around and partially sunk into an outer periphery of the electrotherapeutic device assembly guide 16 and running in parallel tracks from the proximal end 12 to shortly before the distal end 11. In addition, the electrotherapeutic device assembly guide 16 has a central bore/delivery channel 20 providing a fluid channel and/or a working channel for surgical instruments. The outer periphery of the electrotherapeutic device assembly guide 16 fits within the lumen of the outer tube.
- Eight electrotherapeutic device assemblies each comprising a cylindrical guide sheath 30. The guide sheaths 30 are preferably formed in a thermoplastic elastomer or a similar electrically insulating material, and are received in the straight semi-open channels 17 in the electrotherapeutic device assembly guide 16 and firmly attached therein. The cylindrical guide sheaths 30 have a flattened proximal 32 and distal end 31. The interior of each electrotherapeutic device assembly guide sheaths 30 comprises four mutually electrically insulated electrotherapeutic device channels 50 running in parallel from the proximal 32 to the distal 31 end, and distributed in a pattern that resembles a square with the electrotherapeutic device channels 50 placed in the corners. The proximal end of each electrotherapeutic device channel 50 comprises an electrotherapeutic device support zone with a slightly increased diameter for the first approximately 20 mm, to receive a corresponding supporting sheath that is mounted on the proximal end 62 of each electrotherapeutic device 60. Further, the electrotherapeutic device assemblies comprise a total of thirty-two elongate, preferably cylindrical electrotherapeutic devices 60 formed in an electrically conductive material such as titanium, stainless steel or the like, each electrotherapeutic device having proximal ends 62, terminal tips 61 and intermediate main body parts 63. Approximately 20 mm from the proximal 62 end of each electrotherapeutic device 60, a supporting sheath (not shown) 20 mm long may be provided, the sheath surrounding a part of the main body part 63 of the electrotherapeutic device 60. This supporting sheath is meant to lend support to the individual electrotherapeutic devices to prevent buckling or bending during the deployment sequence and is configured to slide into the electrotherapeutic device support zone (of the electrotherapeutic device channels 50 on the guide sheaths 30) when the electrotherapeutic device is moved from its retracted to its advanced position during deployment. Each electrotherapeutic device 60 is preferably covered with an electrically insulating layer except on the distal tip which is left without insulation. Yet further, the electrotherapeutic devices 60 are grouped in groups of four, and each group of electrotherapeutic devices is inserted in a cylindrical guide sheath 30, one electrotherapeutic device in each electrotherapeutic device channel 50. Insertion is done so that the proximal ends 62 of the electrotherapeutic devices 60 protrude approximately 30 mm from the proximal ends of the guide sheaths 30, whereas the terminal tips 61 of the electrotherapeutic devices 60 protrude approximately 40 mm from the distal ends of the guide sheaths 30.
- Eight alignment bushings 80, each configured to receive and guide four electrotherapeutic devices 60 and each with a proximal end 82 and a distal end 81 and four alignment channels 83. The alignment bushings 80 are placed in extension of each of the eight electrotherapeutic device assemblies (guide sheaths 30), and are configured to interface with said assemblies and guide sheaths 30 and to receive the four elongate electrotherapeutic devices 60 where they emerge from the distal ends 31 of said assemblies/guide sheaths 30 in a manner to prevent electrotherapeutic device buckling or bending during the deployment sequence. To achieve this, the proximal end 82 of each alignment bushing 80 is configured to align the four alignment channels 83 with the four electrotherapeutic device channels 50 of the electrotherapeutic device assemblies/guide sheaths 30. The path of the alignment channels 83 of each alignment bushing 80 is configured to change the pattern of the elongate electrotherapeutic devices from the square pattern configuration when emerging from the electrotherapeutic device assemblies/guide sheaths 30 to a linear pattern when they emerge from the alignment bushing 80. Since the eight electrotherapeutic device assemblies/guide sheaths 30 are distributed in a circular pattern and the eight alignment bushings 80 are placed in extension of the assemblies, a radial pattern may be created by suitably orienting the alignment bushings 80.
- A distal tip 13 that is an immediate extension of, and aligned with, the electrotherapeutic device assembly guide 16. The distal tip 13 comprises eight elongate, roughly triangular spacer units 40, each with a proximal end 42 and a tapered, rounded distal end 41, a rounded outer surface 43 and an inner section with two faces 44a, 44b. One face 44b is smooth and one face 44a comprises four distributor grooves 70 that run from the proximal end 42 towards the distal end 41 while curving towards the outer rounded surface 43 of the spacer unit 40, each in a predetermined unique curve. The faces 44a, 44b meet in a 45 degree angle to create a wedge. A rounded cut-out 45 takes away the sharpened end of the wedge. The proximal ends 42 of the spacer units 40 have a reduced height and are inserted into the distal end 11 of - and held tightly together by - the outer tube 15 while the distal ends 41 of the spacer units 40 meet to form a torpedo-shaped tip 13. When all eight wedge-shaped spacer units 40 are held together by the outer tube 15, the rounded cut-outs 45 create a central bore 46 aligned with the delivery channel 20 of the electrotherapeutic device assembly guide 16. The spacer units 40 are oriented so that the smooth face 44b of one spacer unit 40 rests against the face 44a comprising four distributor grooves 70 of the neighbouring spacer unit 40, thus creating four distributor channels 70 per spacer unit 40, for a total of 32 channels. Each distributor channel 70 is configured to receive a specific elongate electrotherapeutic device 60 where it emerges from its respective alignment bushing 80 and to permit its longitudinal movement between a first retracted and a second advanced position (in the same manner as shown in Figs. 13 and 14 respectively). In their first retracted positions, all electrotherapeutic devices 60 are placed with their terminal tips 61 entirely within the distributor channels 70. When the electrotherapeutic devices are advanced as part of a deployment sequence, the distal ends 61 of the electrotherapeutic devices 60 are moved out of the distributor channels 70 to protrude from the distal tip 13. As the grooves and thus the channels 70 lead towards the rounded outer surface 43 of each spacer unit 40 (and thus are deflected away from the longitudinal axis of the introducer shaft 10) and each in its own angle, each electrotherapeutic device is given its own path and emerges from the distal tip 13 in its own direction when advanced. Thus, by providing 32 electrotherapeutic devices that may be moved between a first retracted and a second advanced position, each with a unique path that leads away from the distal tip 13 and ends in a unique point it is possible to generate a three-dimensional pattern of electrotherapeutic device points 60 as previously described.
- A round adaptor plate 90, fixedly attached to the proximal ends 62 of the elongate electrotherapeutic devices 60 and placed proximally to the proximal end of the electrotherapeutic device assembly guide 16. The adaptor plate 90 is longitudinally movable between a first retracted and a second advanced position. The proximal ends 62 of the elongate electrotherapeutic devices are inserted in holes 92 in the adaptor plate 90 that are placed in a pattern resembling that of the electrotherapeutic devices 60 when they emerge from the guide sheaths 30 and the supporting sheath of each electrotherapeutic device is fixedly attached to the adaptor plate 90. The adaptor plate 90 further comprises a central hole 93 that is aligned with the delivery channel 20 of the electrotherapeutic device assembly guide 16, as well as two guide pins 91 that are placed oppositely to each other on - and protruding from - the outer periphery of the adaptor plate 90.

The handle section 100 comprises the following:
- A generally cylindrical housing 101 that is preferably formed in plastic or another suitable material. The housing comprises two half sections, each having an inner and an outer surface, a proximal end, a distal end and an intermediate zone.
- A deployment slider 102 that is preferably made of plastic or a similar non-conductive material and is movable between a first retracted and a second advanced position within and relative to said housing 101. The deployment slider 102 has a proximal end 104 and a distal end 104 and is in operative connection with the adaptor plate by means of two connecting clamps 105. Said connecting clamps 105 are configured to engage the guide pins 91 of the adaptor plate 90 and are slidably held in grooves 109 in the housing 101. The distal end of the deployment slider comprises 32 connections 106 that are configured to receive the proximal ends 62 of the electrotherapeutic devices 60 as they emerge from the adaptor plate 90. Said connections 106 are electrically connected to the terminal tips of flexible leads (not shown) that conduct electric pulses from the electric stimulus generator 120 to the electrotherapeutic devices 60. The proximal ends of said leads are connected to a connector plug that constitutes an interface to an electric stimulus generator 120. The deployment slider 102 further comprises a central bore 107 aligned with the central hole 93 in the adaptor plate 90, as well as two or more finger grips 108 that protrude radially away from the outer surface of the housing 101, through openings in the same. Said finger grips 108 permit an operator to move the deployment slider 102 between a first retracted position and a second advanced position, in order to advance the electrotherapeutic devices 60. The distal half ends of the housing 101 are fixedly attached to the introducer shaft 10 so that the proximal part of the shaft 10, as well as the adaptor plate 90 and the deployment slider 102, all lie within the housing 101. Towards the distal part of the inner surface of each half section of the housing 101 is a groove 109 that is configured to receive one of two connecting clamps 105 of the deployment slider 102. In a proximal continuation of said groove 109 is placed a motion control slot 112 (see Fig. 10) that runs to the proximal end of each half section. The motion control slot 112 is configured to receive one of two finger grips 108 of the deployment slider 102 and permit longitudinal motion of the slider 102 between a first retracted and a second advanced position. The proximal end of the housing 101 is threaded to receive an end cap 110 that serves the dual purpose of closing the handle section 101 and holding the proximal ends of the two half sections of the housing 101 together. Further, one half section comprises an outlet configured to receive the leads 121, 122 as they emerge from the deployment slider 102.
- An end cap 110 that comprises an outer shell with a threading on its inner surface and an inner support cylinder that has a circumference corresponding with the circumference of the inner surface of the housing. The end cap further comprises a central hole 111 that is aligned with the central bore in the deployment slider 102 and is configured to receive the tubing of the drug dispenser.

In use, the connector plug of the device is connected to a suitable electric stimulus generator 120. The device 1 is then inserted through a bore hole in the patient's skull and introduced to the target region of the patient's body/brain. The precise location may be identified by means of ultrasound, CT, MR or another suitable means, and the correct position of the introducer shaft 10 prior to deployment may be verified by similar means. As described above, in other embodiments, the stimulus generator may be integrated in the handle section.

When a correct position of the introducer shaft 10 has been obtained, an operator may deliver a suitable chemotherapeutic agent or dose of genetic material through the central channel 111, 107, 93, 20 and into the tissue region to be treated. Delivery is done by inserting the elongate, length-adjusted and properly dulled needle of a syringe 115 in the central hole of the end cap and advancing it until no further motion is possible. The operator may then empty the syringe barrel 115 by pressing the syringe plunger, whereupon the liquid in the syringe is expelled into the tissue to be treated.

Before, during or upon delivery, the operator may deploy the elongate electrotherapeutic devices 60 in a predefined pattern. Deployment is done by moving the deployment slider 102 from its first retracted position towards its second advanced position until further movement is prevented by the end of the motion control slots 112. Said movement results in the motion of the electrotherapeutic devices 60 from the first retracted to the second advanced position. The distributor channels 70 in the distal tip 13 are shaped to provide each individual electrotherapeutic device 60 with a unique, preferably essentially linear path through the tissue and a unique end-point, and the goal is to enable the creation of an electrotherapeutic device pattern that may have a larger diameter (or maximum extent in a plane perpendicular to the longitudinal axis of the shaft 10) than the introducer shaft 10 and may ensure optimal distribution of the short and intense pulses and the thereby derived electric fields in the tissue to be treated. In one particular preferred embodiment the un-insulated electrotherapeutic device tips (terminal tips 61) are positional and positioned with their end-points at least partially surrounding or enclosing the target region of tissue in such a way that the terminal tips 61 describe or define the outer periphery of a spherical/spatial ellipse. In said preferred embodiment the 32 electrotherapeutic devices are organized in four layers, each layer having a different diameter and consisting of eight electrotherapeutic devices 60 with their end-points (terminal tips 61) describing a circular pattern in a plane perpendicular to the axis of the introducer shaft 10.

Upon deployment, an operator may activate the electric stimulus generator 120 to deliver a sequence of preferably short and intense electric pulses, for example square-wave pulses, to the tissue to be treated. To ensure a suitable distribution of pulses and the consequent electric fields in the tissue to be treated (target tissue), pulses may be assigned to alternating specific electrotherapeutic devices 60 in a pattern that may be tailored to suit the anatomy of the individual region of the body to be treated and/or the geometry of the specific malignant target tissue. In an embodiment, at least some of the end-points 61 of the electrotherapeutic devices 60 are placed in equidistant relation to other electrotherapeutic device end points 61, and at least some pulses are assigned to equidistant pairs of electrotherapeutic devices. Thus, a homogenous or heterogeneous, controllable three-dimensional electric field can be created in the target tissue.

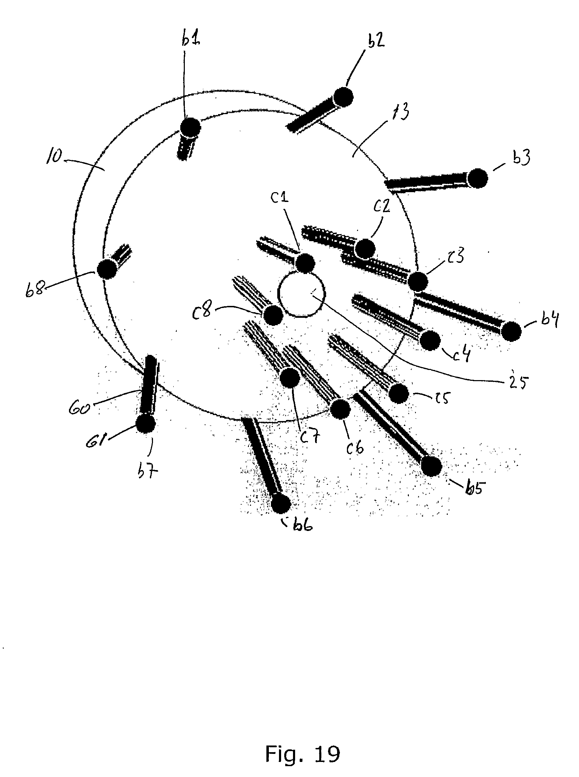

In a further embodiment the un-insulated electrotherapeutic device 60 tips are positionable in such a pattern that their end-points 61 outline an outer periphery of an ellipsoid or an ellipse in a plane taken parallel to the longitudinal axis of the shaft 10 - corresponding to what is illustrated by reference E in Fig. 15. In this embodiment, and as further shown in Fig. 19, the 32 electrotherapeutic devices 60 are organized in four substantially parallel layers (in a plane perpendicular to the longitudinal axis of the shaft 10) numbered a-d, (a being the top-most (with respect to the distal tip 13)/most-distal layer (with respect to the user/surgeon)) consisting of eight electrotherapeutic devices numbering 1-8 in each layer, with their end-points describing an elliptical or a circular pattern perpendicular to the axis of the introducer shaft. In Fig. 19, the top layer a and bottom layer d of electrotherapeutic devices 60 has been left out, for the purpose of clarity, such that the b (b1-b8) and c (c1-c8) layers are shown.

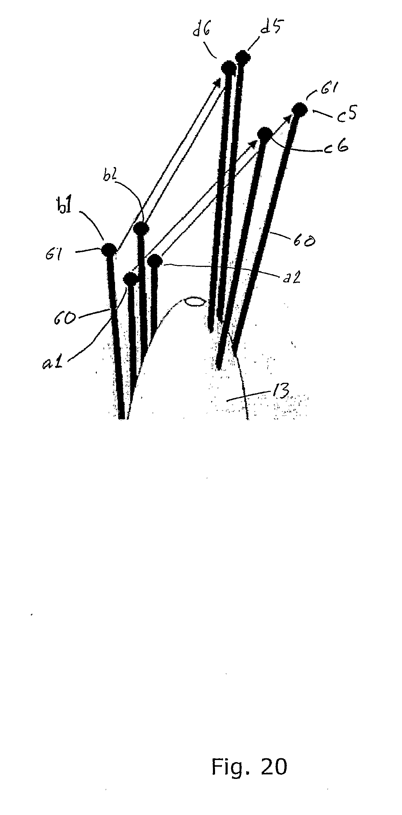

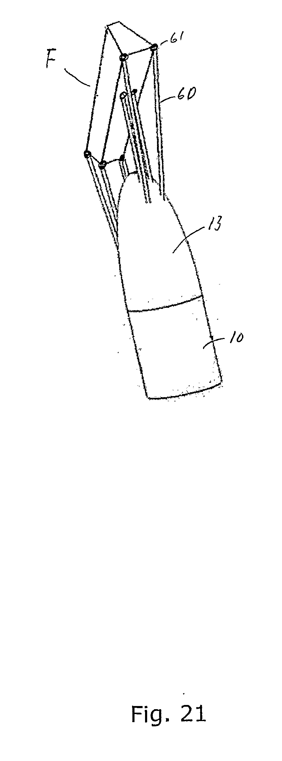

The efficiency of the electroporation may be enhanced by adapting a controlled pulse emitting sequence, thus creating a controlled electric field. In one suggested pulse sequence, at least some of the pulses assigned travel from electrotherapeutic devices in layer a to electrotherapeutic devices in layer c that are placed in equidistant relation to the electrotherapeutic devices in layer a, while others simultaneously travel between equidistant pairs in layer b and layer d. In one particular firing sequence, pulses travel from positive electrotherapeutic devices a1 and a2 to negative electrotherapeutic devices c6 and c5, and simultaneous pulses travel from positive electrotherapeutic devices b1 and b2 to negative electrotherapeutic devices d6 and d5, as illustrated in Fig. 20 where only the mentioned electrotherapeutic device terminal tips 61 are shown, the other 24 being removed for the sake of clarity. The pulses will travel the shortest possible way (assuming uniform electric resistance in the target tissue) wherefore the electric field can be shaped and controlled by the positioning of the electrotherapeutic devices such that firing between the electrotherapeutic devices in different layers can be made between equidistant positive and negative pairs of electrotherapeutic device ends (61) (point electrotherapeutic devices). Thus, an elongate, three-dimensional electric field F is generated, as shown in Fig. 21. The position of the field may be altered to cover the largest possible tissue volume by sequentially changing the assignment of pulses to other equidistant positive and negative electrotherapeutic devices in a suitable pattern.

Upon pulse delivery, the operator may retract the elongate electrotherapeutic devices 60 to their first retracted position by moving the deployment slider 102 from the second advanced position to the first retracted position whereby the electrotherapeutic devices are retracted to their default position within the distal tip 13, and the device 1 may be removed from the body of the patient. Alternatively, the operator may reposition the device after having retracted the elongate electrotherapeutic devices 60, potentially permitting multiple pulse applications covering a larger area in a single device insertion.

In either of the above embodiments a separate channel (not shown) or a portion of the delivery channel 20 may be used to deliver a saline solution to enhance the Electroporation process by increasing tissue conductivity. A saline solution may also be introduced via the delivery channel 20 proper. In either case suitable means for connecting the channel 20 to a source of saline solution may preferably be provided at the handle section.100

As described above, the cross-sectional shape of the electrotherapeutic devices is preferably essentially circular. However, in other embodiments, other cross sectional shapes may be applied. The diameter and cross-sectional shape of the distributor channels 70 are in any event preferably dimensioned for the desired electrotherapeutic device diameter and cross-sectional shape, in order to provide the best possible support for the electrotherapeutic devices, without limiting their ability to be moved from their retracted position to their extended position (and back).

In either of the above described embodiments, the electrotherapeutic device diameter is preferably 0.4 mm or smaller, such as 0.3 mm, 0.25 mm including electrically insulating coating. The diameter of the electrotherapeutic devices 60 is typically correlated to the stiffness of the electrotherapeutic devices, such that the thicker the electrotherapeutic device, the stiffer the electrotherapeutic device. For some applications a stiff electrotherapeutic device may be necessary, e.g. if the tissue is tough. In soft tissue a less stiff electrotherapeutic device may be applied.

Also depending on the application, the tip of the electrotherapeutic devices may be configured such that it may cut through tissue or it may be smooth in order to more gently spread the tissue.

Further, the electrotherapeutic devices may biased (e.g. pre-tensioned) in such a way that their geometrical configuration in their extended state varies with the extent to which they have been extended beyond the distal tip 13 of the shaft 10. This may be applied be providing the electrotherapeutic devices 60 with different tension characteristics along the lengthwise direction of the electrotherapeutic devices. Thus, a very flexible electroporation device may be obtained.

In the description above and in the drawings, the delivery channel 20 has been illustrated to be centrally located within the shaft 10. However the delivery channel 20 may be asymmetrically located within the shaft, with respect to its cross sectional position. In other embodiments (not shown) the single delivery channel 20 may be replaced by a plurality of smaller delivery channels, each having an outlet at the tip 13. Thereby a more even distribution of an injected therapeutic molecule solution can be obtained.

As described above, a surgical tool or the like may be inserted via the delivery channel 20. The invention also concerns a combination of an electroporation device having a delivery channel according to any of the embodiments described above and an therapeutic molecule solution injection device. The therapeutic molecule solution injection device comprises an elongate hollow part adapted for the delivery channel 20, and a steerable outlet tip. The elongated hollow part is adapted in length, such that the steerable outlet tip can be extended beyond the tip 13 of the electroporation device. The steerable outlet tip may be used to administer a dose of therapeutic molecule solution in a precise location in the target tissue.

Alternatively, or in addition to the combination with therapeutic molecule solution injection device, the electroporation device may have a steerable tip 13. This may be provided by having control rods or strings extending through the shaft 10 to the tip 13, the tip e.g. being pivotally mounted at the distal end of the shaft 10, pivotably about an axis either parallel to the elongate axis of the shaft or perpendicular (or at another angle) to the axis of the shaft. The extent to which the tip 13 may be steered is of course dependant on the stiffness of the electrotherapeutic devices, and a flexible alignment between the channels 50 in the shaft and the channels 70 in the tip 13. By providing a steerable tip 13, the flexibility and reach of the electroporation device may be enhanced, since for also a larger target tissue volume, a single entry hole/channel, formed by the shaft 10 through the surrounding (healthy) tissue is necessary. Thus the reach of the electrotherapeutic devices may be expanded by a turning of the tip 13 or a combination of a turning of the shaft and a tipping of the tip 13 (when the electrotherapeutic devices are in retracted position in the shaft) Thereby the applied electrical field can be repositioned, in a sequence until the entire target tissue may be covered. Further the direction of the outlet of the delivery channel may be altered in order to provide for a more precise delivery of a therapeutic molecule solution. The steerable tip 13 may be combined with the above mentioned therapeutic molecule solution injection device in order to further enhance the reach and flexibility of the drug delivery. However, the steerable tip 13 may also be applied in embodiments without a delivery channel, i.e. embodiments suitable for systemic introduction of drugs or for irreversible electroporation.

The electrotherapeutic devices may also be prepared with/covered by/impregnated with a drug or DNA molecule compound that may be dissolvable in an electrical field. Thereby, a drug etc. may be released from the electrotherapeutic devices when an electrical field is applied to the target tissue via the electrotherapeutic devices. Thereby the delivery channel 20 may be spared. However, the drug impregnated electrotherapeutic devices may also be used with embodiments having a delivery channel 20 in order to release multiple drugs or in order to save the delivery channel for e.g. a field enhancing saline solution as described above.

In embodiments of the invention wherein said terminal tips 61 of said electrotherapeutic devices 60 are deflectable away from a longitudinal axis L of said shaft 10 when deployed/extended to their extended position, such that at least one planar projection taken in a plane perpendicular to said longitudinal axis L of a distance D1 between at least one pair of terminal tips 61 of said electrotherapeutic devices 60 is larger than a maximal extent D2 of a cross-section of said introducer shaft 10 , said cross-section taken in a plane perpendicular to said longitudinal axis L at a distal end 12 of said introducer shaft 10 , the deflection of said terminal tips 61 of said electrotherapeutic devices 60 , when in their extended position, may alternatively be provided by a biasing of at least a section of said main body part 63 of said electrotherapeutic devices 60. In further embodiments the distal tip 13 of the introducer shaft 10 may be formed with a substantially smooth, rounded, non-cutting shape with a substantially smooth, non-cutting transition to the introducer shaft 10 . In further embodiements, said distal tip 13 may be detachable from said introducer shaft 10. In further embodiements, said electrotherapeutic devices 60 may be extended individually or in sets. In further embodiements of all previously described embodiments, said electrotherapeutic devices 60 may be extendable such that their terminal tips 61 form a spatial distribution around a volume of tissue. Thus, in further embodiements, said electrotherapeutic devices 60 may be extendable such that their terminal tips 61 form a substantially spatial spherical or ellipsoid distribution pattern, having a circular or elliptical cross section E taken in a plane parallel to said longitudinal axis L when extended.

In further embodiements, where said terminal tips 61 are enclosed within an introducer shaft 10 , said electrotherapeutic devices 60 may be slideably arranged in electrically insulated guide channels 50, 70 formed in the introducer shaft 10 and/or distal tip 13 of the introducer shaft. In further embodiemtents, said introducer shaft 10 may further comprise a fluid delivery channel 20 through which a dose of therapeutic molecules can be administered, said fluid delivery channel 20 extending through the length of said introducer shaft 10 and terminating through said distal tip 13 , said fluid delivery channel 20 being separate from said distributor channels 70 . In further embodiements, the electroporation device 1 may comprise a handle section 100 , said elongate introducer shaft 10 extending from said handle section 100 . The handle section 100 may comprise an energy source for applying through said electrotherapeutic devices 60 an electrical field to a target tissue, when the electrotherapeutic devices 60 are in their extended position. In a further embodiment, the handle section 100 may comprise a therapeutic molecule delivery system comprising a therapeutic molecule reservoir and actuating means for administering said therapeutic molecules through said fluid delivery channel 20 .

In further embodiements, where said terminal tips 61 are enclosed within an introducer shaft 10 , said introducer shaft 10 may have a circular cross section with an outer diameter D2 of 10 mm or less, preferably of 5 mm or less, more preferably of 3.5 mm or less. In further embodiements, the introducer shaft 10 may comprise an outer tube 15 and an inner electrotherapeutic device assembly guide 16 received in said outer tube 15 , and where said electrotherapeutic devices 60 are slideably arranged in guide channels 50 formed in said inner electrotherapeutic device assembly guide 16 . In further embodiements, said guide channels 50 may be formed in a set of cylindrical guide sheaths 30 that are received in longitudinal semi-open channels 17 distributed radially along the periphery of said inner electrotherapeutic device assembly guide 16 .

As a further embodiement of all the above described embodiements each electrotherapeutic device 60 of the electroporation device 1 may be assigned an individual electric polarity, such that the electric stimuli can be provided from and between individual electrotherapeutic devices 60.

In a further embodiment, where the terminal tips 61 are located at an outer surface of said distal tip 13 of said introducer shaft 10 when in the retracted position and where the distal tip is covered by a dissolvable layer, said dissolvable layer may be gradually dissolvable by contact to internal tissue of a patient or is dissolved by application of a suitable energy from the electroporation device 1 itself, or from an external source.

In all of the above described embodiements the terminal tips 61 of said electrotherapeutic devices 60 may further have a smooth, rounded outer surface geometry. In further embodiments thereof said terminal tips 61 may be elliptical, or the tips 61 may alternatively be substantially spherical.

In a further embodiment of any of the above described embodiments, a transitional surface 65 from the elongate main body part 63 to the terminal tip 61 of at least one electrotherapeutic device 60 is smooth and rounded.

In further a embodiment of any of the above described embodiments, at least one of said elongate main body parts 63 of the electrotherapeutic devices 60 may have a circular cross sectional shape, A1, in a direction perpendicular to said longitudinal axis, B.

In further a embodiment of any of the above described embodiments, said second electrically conductive surface 161 is electrically connected to said first electrically conducting path 66.

In further a embodiment of any of the above described embodiments, wherein at least one of the electrotherapeutic devices 60 has a second electrically conductive surface 161, the second electrically conductive surface 161 may be electrically connected to a second electrically conducting path 166 extending along said elongate main body parts 63 from said proximal end 62 to said second electrically conductive surface 161, said second electrically conductive path 166 being electrically insulated from said first electrically conductive path 66. In further a embodiment thereof, said second electrically conductive surface 161 may circumscribe the elongate main body part 63. In a further embodiment said second electrically conductive surface 161 may show a substantially rectangular cross sectional shape in a cross section parallel to said longitudinal axis B. Alternatively, an outer contour of said second electrically conductive surface 161 may define a convex arc in a cross section parallel to said longitudinal axis B. Thus, in one embodiment, an outer contour of said second electrically conductive surface 161 may define a semi-circle in a cross section parallel to said longitudinal axis B. In a further embodiment of any of the above described embodiments, a transition from the outer surface of said elongate main body part 63 to the second electrically conductive surface 161 is smooth and rounded. In a further embodiment of any of the above described embodiments the second electrically conducting path 166, extending along said elongate main body part 63, to said second electrically conductive surface 161 is a tubular structure being electrically insulated from said first electrically conducting path 166. In a further embodiment said first electrically conducting path 66 may be centrally structured within said main body part 63. In a further embodiment of any of the above described embodiments, said first electrically conducting path 66 is a monofile wire.

In a further embodiment of any of the above described embodiments, wherein at least one main body part 63 of an electrotherapeutic device 60 has a fluid passage 68 extending in the direction of said longitudinal axis B, said fluid passage 68 may be centrally located within said electrotherapeutic device 60, and at least one of said first or second electrically conducting paths 66, 166 may be a tubular conductor 166 formed concentrically around said fluid passage 68.

In a further embodiment of any of the above described embodiments, wherein said elongate main body parts 63 further comprise an outermost non-conductive, electrically insulating layer 67, the outermost non-conductive, electrically insulating layer 67 may have a width of less than 1 mm, preferably less than 0.5 mm, more preferably less than 0.25 mm, and even more preferably less than 0.10 mm, and even more preferably substantially 0.025 mm.

In a further embodiment of any of the above described embodiments, wherein at least one distributor channel 70 has a linear section 71 provided distally to a curved section 72 such that the path of an electrotherapeutic device 60 deployed to its extended position is substantially linear, at least one electrotherapeutic device 60 may be extendable to a position extending beyond the distal-most end of the distal tip 13 of the introducer shaft 10. In a further embodiment at least one terminal tip 61 of an electrotherapeutic device 60 may be extendable to a position extending beyond the distal-most end of the distal tip 13 of the introducer shaft 10.

The device according to the invention may be used in an electroporation method for creating one or more electrical fields to generate an electroporation and/or electrophoretic effect in a target tissue in the body of a patient, comprising the steps of
• providing an electroporation device 1 comprising
   ∘ an elongate introducer shaft 10, said introducer shaft 10 having a distal tip 13; and
   ∘ a plurality of electrotherapeutic devices 60 having respective distal ends 64, wherein at least one of said insertable electrotherapeutic devices 60 comprises
      ▪ an elongate main body part/wire section 63 having a centrally located longitudinal axis B, a distal end 64 and a proximal end 62;
      ▪ an electrically conductive terminal tip 61 disposed at said distal end 64 of said elongate body part 63; and
      ▪ a first electrically conducting path 66 extending along said elongate main body part 63 from said proximal end 62 to said distal end 64,
      ▪ wherein said elongate main body part 63 is of substantially uniform cross sectional area A1, and wherein a maximal cross sectional area A2 of said terminal tip 61 in a direction perpendicular to said longitudinal axis B is greater than the cross sectional area A1 of said elongate main body part 63
      each electrotherapeutic device 60 being slidably arranged within said introducer shaft 10 from a retracted position, where said terminal tips 61 are enclosed within said introducer shaft 10 or distal tip 13 or located adjacent to said distal tip 13, to an extended position, where said terminal tips 61 extend from said distal tip 13 to a plurality of laterally extending angularly spaced positions,
• inserting said introducer shaft 10 through tissues of a body and bring said distal tip 13 into a vicinity of a target region to be treated, while said electrotherapeutic devices 60 are in said retracted position;
• extending said electrotherapeutic devices 60 to said extended position to at least partially surround tissue in a target region of the patient; where the terminal tips 61 create a virtual, three-dimensional enclosure of finite points to partially or fully enclose said target tissue; and
• Transmitting from one or more electrotherapeutical devices 60 to one or more different electrotherapeutical devices 60 one or more electric pulses of specific amplitudes and durations to create one or more electric fields in said target tissue.

In this method said transmission may further comprise assigning, sequentially and selectively through one or more electrotherapeutic devices 60 electrical pulses of given amplitudes and durations, and assigning sequentially and selectively to one or more different electrotherapeutic devices 60 electric pulses of an opposing electrical polarity to create simultaneously and/or sequentially one or more electric fields in said target tissue. Yet further the method may comprise extending the electrotherapeutic devices 60 such that their terminal tips 61 form a spatial distribution at least partly around a volume of tissue. Yet further the method may comprise extending the electrotherapeutic devices 60 individually or in sets to their extended positions to a spatial configuration of terminal tips 61 at least partially surrounding a target tissue. Yet further the method may comprise extending the electrotherapeutic devices 60 such that their terminal tips 61 form a substantially spherical distribution pattern. Yet further the method may comprise a step of administering a dose of therapeutic molecules to said body prior to, while or after applying through said electrotherapeutic devices 60 said pulses. In one variation of this method the said dose may be administered locally in the vicinity of the target region, through a fluid delivery channel 20 , said delivery channel 20 extending through the length of said shaft 10 and terminating through said distal tip 13 , said fluid delivery channel 20 being separate from distributor channels 70 arranged in at least the distal tip of the introducer shaft 10 , said distributor channels 70 providing a deflection of said terminal ends 61 of said electrotherapeutic devices 60, when in their extended position.

The device according to the invention may be used in an electroporation method for creating one or more electrical fields to generate an electroporation and/or electrophoretic effect in a target tissue in the body of a patient, comprising the steps of
• providing an electroporation device 60 comprising a plurality of electrotherapeutic devices 60 having respective distal ends 64 , each electrotherapeutic device 60 being slidably arranged with respect to a reference point P from a retracted position, to an extended position, where said distal end 64 extends distally beyond the position of said distal end 64 when in said retracted position, wherein at least one of said electrotherapeutic devices 60 comprises
   ∘ an elongate main body part/wire section 63 having a longitudinal axis B, a distal end 64 and a proximal end 62;
   ∘ an electrically conductive terminal tip 61 disposed at said distal end 64 of said elongate body part 63; and
   ∘ a first electrically conducting path 66 extending along said elongate main body part 63 from said proximal end 62 to said distal end 64,
   wherein said elongate main body part 63 is of a substantially uniform cross sectional area A1, and wherein a maximal cross sectional area A2 of said terminal tip 61 in a direction perpendicular to said longitudinal axis B is greater than the cross sectional area A1 of said elongate main body part 63;
• inserting the terminal tips into the body of a patient at a desired location, and extending said electrotherapeutic devices 60 to said extended position to at least partially surround tissue in a target region of the patient; where the terminal tips 61 create a virtual, three-dimensional enclosure of finite points to partially or fully enclose said target tissue; and
• Transmitting from one or more electrotherapeutical devices 60 to one or more different electrotherapeutical devices 60 one or more electric pulses of specific amplitudes and durations to create one or more electric fields in said target tissue.

In a variation of this method, the transmission may comprise assigning, sequentially and selectively through one or more electrotherapeutic devices 60 electrical pulses of given amplitudes and durations, and assigning sequentially and selectively to one or more different electrotherapeutic devices 60 electric pulses of an opposing electrical polarity to create simultaneously and/or sequentially one or more electric fields in said target tissue.

Although the present invention has been described in connection with the specified embodiments, it should not be construed as being in any way limited to the presented examples. The scope of the present invention is set out by the accompanying claim set. In the context of the claims, the terms "comprising" or "comprises" do not exclude other possible elements or steps. Also, the mentioning of references such as "a" or "an" etc. should not be construed as excluding a plurality. The use of reference signs in the claims with respect to elements indicated in the figures shall also not be construed as limiting the scope of the invention. Furthermore, individual features mentioned in different claims, may possibly be advantageously combined, and the mentioning of these features in different claims does not exclude that a combination of features is not possible and advantageous.

## Claims

1. An electroporation device (1) for inducing an electrical field in the body of a patient comprising a plurality of electrotherapeutic devices (60) having respective distal ends (64), each of the electrotherapeutic devices (60) being slidably arranged with respect to a reference point (P) of the electroporation device (1) from a retracted position, to an extended position, where said distal end (64) of each of the electrotherapeutic devices (60) extends distally beyond the position of said distal end (64) when in said retracted position, wherein at least one of said electrotherapeutic devices (60) comprises
• an elongate main body part (63) having a longitudinal axis (B), a distal end (64) and a proximal end (62);
• an electrically conductive terminal tip (61) disposed at said distal end (64) of said elongate body part (63); and
• a first electrically conducting path (66) extending along said elongate main body part (63) from said proximal end (62) to said distal end (64),
**characterized in that** said elongate main body part (63) is of a substantially uniform cross sectional area (A1) along said longitudinal axis (B), wherein a maximal cross sectional area A(2) of said terminal tip (61) in a direction perpendicular to said longitudinal axis (B) is greater than the cross sectional area (A1) of said elongate main body part (63) and wherein said terminal tip (61) of said electrotherapeutic devices (60) has a smooth, rounded outer surface geometry.

2. An electroporation device (1) according to claim 1 comprising an elongate introducer shaft (10), said introducer shaft (10) having a distal tip (13); said plurality of electrotherapeutic devices (60) being slidably arranged within said introducer shaft (10) from a retracted position, where said terminal tips (61) are enclosed within said introducer shaft (10) or fully or partially within said distal tip (13) or located at an outer surface of said distal tip (13), to an extended position, where said terminal tips (61) extend from said distal tip (13) to a plurality of laterally extending angularly spaced positions.

3. An electroporation device (1) according to claim 2, wherein said terminal tips (61) of said electrotherapeutic devices (60) are deflectable away from a longitudinal axis (L) of said shaft (10) when deployed/extended to their extended position, such that at least one planar projection taken in a plane perpendicular to said longitudinal axis (L) of a distance (D1) between at least one pair of terminal tips (61) of said electrotherapeutic devices (60) is larger than a maximal extent (D2) of a cross-section of said introducer shaft (10), said cross-section taken in a plane perpendicular to said longitudinal axis (L) at a distal end (12) of said introducer shaft (10).

4. An electroporation device (1) according to claim 3, wherein the deflection of said terminal tips (61) of at least one of said electrotherapeutic devices (60), when in their extended position, is provided by a curving of a distributor channel (70) provided in at least said distal tip (13) of the introducer shaft (10).

5. An electroporation device (1) according to any of claims 2-4, wherein the terminal tips (61) are enclosed within the distal tip (13) when in their retracted position.

6. An electroporation device (1) according to claim 5 wherein at least one terminal tip (61) is hidden within an enlargement (73) formed in the distal-most end of the distributor channels (70) provided in said distal tip (13), when in their retracted position.

7. An electroporation device (1) according to any of claims 2-4 wherein said terminal tips (61) are located at an outer surface of said distal tip (13) of said introducer shaft (10) when in the retracted position and where the distal tip is covered by a dissolvable layer, such that said terminal tips (61) are enclosed in said dissolvable layer, when in retracted position, and such that said dissolvable layer forms a substantially smooth, rounded, non-cutting shape with a substantially smooth, non-cutting transition to the introducer shaft (10).

8. An electroporation device (1) according to any of claims 1-7 wherein said terminal tip (61) and said electrically conducting path (66) are formed in different electrically conductive materials.

9. An electroporation device (1) according to any of claims 1-8 wherein said elongate main body part (63) constitutes said first electrically conducting path (66).

10. An electroporation device (1) according to any of claims 1-9 wherein said elongate main body parts (63) further comprise an outermost non-conductive, electrically insulating layer (67).

11. An electroporation device (1) according to claim 10 wherein at least one of said electrotherapeutic devices (60) has a portion of said elongate main body part (63) that is electrically un-insulated to have a second electrically conductive surface (161).

12. An electroporation device (1) according to claim 11, wherein said second electrically conductive surface(s) (161) includes a point with a distance (d3) from and in a direction perpendicular to said central longitudinal axis (B) greater than the maximum distance (d2) from any point on the outer surface of said elongate body part and in a direction perpendicular to said central longitudinal axis (B).

13. An electroporation device (1) according to any of claims 1-12, wherein at least one main body part (63) of an electrotherapeutic device (60) has a fluid passage (68) extending in the direction of said longitudinal axis (B) through which a dose of therapeutic molecules can be administered from a reservoir connected to or connectable to the proximal end (62) of said electrotherapeutic device (60).

14. An electroporation device (1) according to any of claims 1-13 wherein the main bodies (63) of the electrotherapeutic devices (60) are of substantially circular cross-sectional form, and wherein the diameter of said main bodies (63) is less than 2 mm, preferably less than 1 mm, more preferably less than 0.5 mm, and even more preferably less than 0.20 mm.

15. An electroporation device (1) according to any of claims 1-14 wherein the terminal tips (61) of the electrotherapeutic devices (60) has a maximum cross-sectional extent in a direction perpendicular to said axis (B) of less than 3 mm, preferably less than 1,5 mm, more preferably less than 0.75 mm, and even more preferably less than 0.30 mm.

16. An electroporation device (1) according to any of claims 4-15, wherein at least one distributor channel (70) has a linear section (71) provided distally to a curved section (72) such that the path of an electrotherapeutic device (60) deployed to its extended position is substantially linear.

## Patentansprüche

1. Elektroporationsvorrichtung (1) zum Induzieren eines elektrischen Felds im Körper eines Patienten, umfassend eine Mehrzahl von elektrotherapeutischen Vorrichtungen (60) mit jeweils distalen Enden (64), wobei jede der elektrotherapeutischen Vorrichtungen (60) in Bezug auf einen Bezugspunkt (P) der Elektroporationsvorrichtung (1) gleitbar von einer zurückgezogenen Position zu einer ausgezogenen Position angeordnet ist, wobei sich das distale Ende (64) jeder der elektrotherapeutischen Vorrichtungen (60) in der zurückgezogenen Position distal über die Position des distalen Endes (64) hinaus erstreckt, wobei mindestens eine der elektrotherapeutischen Vorrichtungen (60) umfasst
• einen länglichen Hauptkörperteil (63) mit einer Längsachse (B), einem distalen Ende (64) und einem proximalen Ende (62);
• eine elektrisch leitfähige terminale Spitze (61), die an dem distalen Ende (64) des länglichen Körperteils (63) angeordnet ist; und
• einen ersten elektrisch leitenden Weg (66), der sich entlang des länglichen Hauptkörperteils (63) von dem proximalen Ende (62) zu dem distalen Ende (64) erstreckt, **dadurch gekennzeichnet, dass**
der längliche Hauptkörperteil (63) eine im Wesentlichen einheitliche Querschnittsfläche (A1) entlang der Längsachse (B) aufweist, wobei eine maximale Querschnittsfläche A(2) der terminalen Spitze (61) in einer Richtung senkrecht zur Längsachse (B) größer ist als die Querschnittsfläche (A1) des länglichen Hauptkörperteils (63) und wobei die terminale Spitze (61) der elektrotherapeutischen Vorrichtungen (60) eine glatte, gerundete Außenflächengeometrie aufweist.

2. Elektroporationsvorrichtung (1) nach Anspruch 1, umfassend einen länglichen Einführungsschaft (10), wobei der Einführungsschaft (10) eine distale Spitze (13) aufweist; wobei die Mehrzahl der elektrotherapeutischen Vorrichtungen (60) in dem Einführungsschaft (10) gleitbar von einer zurückgezogenen Position, in der die terminalen Spitzen (61) in dem Einführungsschaft (10) oder vollständig oder teilweise in der distalen Spitze (13) eingeschlossen sind oder sich an einer Außenfläche der distalen Spitze (13) befinden, zu einer ausgezogenen Position, in der sich die terminalen Spitzen (61) von der distalen Spitze (13) zu einer Mehrzahl von sich lateral erstreckenden, gewinkelt beabstandeten Positionen erstrecken, angeordnet ist.

3. Elektroporationsvorrichtung (1) nach Anspruch 2, wobei die terminalen Spitzen (61) der elektrotherapeutischen Vorrichtungen (60) beim Einsetzen/Ausziehen in ihre ausgezogene Position von einer Längsachse (L) des Schafts (10) ablenkbar sind, sodass mindestens eine planare Projektion in einer Ebene senkrecht zur Längsachse (L) eines Abstands (D1) zwischen mindestens einem Paar distaler Spitzen (61) der elektrotherapeutischen Vorrichtungen (60) größer ist als eine maximale Ausdehnung (D2) eines Querschnitts des Einführungsschafts (10), wobei der Querschnitt in einer Ebene senkrecht zur Längsachse (L) an einem distalen Ende (12) des Einführungsschafts (10) liegt.

4. Elektroporationsvorrichtung (1) nach Anspruch 3, wobei die Ablenkung der terminalen Spitzen (61) von mindestens einer der elektrotherapeutischen Vorrichtungen (60) in der ausgezogenen Position durch eine Krümmung eines Verteilerkanals (70) in mindestens einer distalen Spitze (13) des Einführungsschafts (10) bereitgestellt ist.

5. Elektroporationsvorrichtung (1) nach einem der Ansprüche 2-4, wobei die terminalen Spitzen (61) in der zurückgezogenen Position in der distalen Spitze (13) eingeschlossen sind.

6. Elektroporationsvorrichtung (1) nach Anspruch 5, wobei mindestens eine terminale Spitze (61) in der zurückgezogenen Position in einer Erweiterung (73) in dem distalsten Ende der Verteilerkanäle (70) in der distalen Spitze (13) ausgebildet ist.

7. Elektroporationsvorrichtung (1) nach einem der Ansprüche 2-4, wobei sich die terminalen Spitzen (61) in der zurückgezogenen Position an einer Außenfläche der distalen Spitze (13) des Einführungsschafts (10) befinden und wobei die distale Spitze von einer löslichen Schicht bedeckt ist, sodass die terminalen Spitzen (61) in der zurückgezogenen Position in der löslichen Schicht eingeschlossen sind und sodass die lösliche Schicht eine im Wesentlichen glatte, gerundete nicht schneidende Form mit einem im Wesentlichen glatten, nicht schneidenden Übergang zum Einführungsschaft (10) ausbildet.

8. Elektroporationsvorrichtung (1) nach einem der Ansprüche 1-7, wobei die terminale Spitze (61) und der elektrisch leitende Weg (66) in unterschiedlichen elektrisch leitfähigen Materialien ausgebildet sind.

9. Elektroporationsvorrichtung (1) nach einem der Ansprüche 1-8, wobei der längliche Hauptkörperteil (63) den ersten elektrisch leitenden Weg (66) darstellt.

10. Elektroporationsvorrichtung (1) nach einem der Ansprüche 1-9, wobei der längliche Hauptkörperteil (63) weiterhin eine äußerste, nicht leitfähige, elektrisch isolierende Schicht (67) umfasst.

11. Elektroporationsvorrichtung (1) nach Anspruch 10, wobei mindestens eine der elektrotherapeutischen Vorrichtungen (60) einen Abschnitt des länglichen Hauptkörperteils (63) aufweist, der elektrisch nicht isoliert ist, um eine zweite elektrisch leitfähige Fläche (161) bereitzustellen.

12. Elektroporationsvorrichtung (1) nach Anspruch 11, wobei die zweite(n) elektrisch leitfähige(n) Oberfläche(n) (161) einen Punkt mit einem Abstand (d3) zu und in einer Richtung senkrecht zu der zentralen Längsachse (B) einschließt/einschließen, der größer ist als der maximale Abstand (d2) von einem beliebigen Punkt auf der Außenfläche des länglichen Körperteils und in einer Richtung senkrecht zu der zentralen Längsachse (B).

13. Elektroporationsvorrichtung (1) nach einem der Ansprüche 1-12, wobei mindestens ein Hauptkörperteil (63) einer elektrotherapeutischen Vorrichtung (60) einen Flüssigkeitsweg (68) aufweist, der sich in der Richtung der Längsachse (B) erstreckt und durch den eine Dosis therapeutischer Moleküle aus einem Behälter verabreicht werden kann, der mit dem proximalen Ende (62) der elektrotherapeutischen Vorrichtung (60) verbunden oder verbindbar ist.

14. Elektroporationsvorrichtung (1) nach einem der Ansprüche 1-13, wobei die Hauptkörper (63) der elektrotherapeutischen Vorrichtungen (60) eine im Wesentlichen kreisförmige Querschnittsform aufweisen und wobei der Durchmesser dieser Hauptkörper (63) kleiner als 2 mm ist, vorzugsweise kleiner als 1 mm, mehr bevorzugt kleiner als 0,5 mm und noch mehr bevorzugt kleiner als 0,20 mm.

15. Elektroporationsvorrichtung (1) nach einem der Ansprüche 1-14, wobei die terminalen Spitzen (61) der elektrotherapeutischen Vorrichtungen (60) eine maximale Querschnittsausdehnung in einer Richtung senkrecht zu der Achse (B) von weniger als 3 mm ist, vorzugsweise weniger als 1,5 mm, mehr bevorzugt weniger als 0,75 mm und noch mehr bevorzugt weniger als 0,30 mm aufweisen.

16. Elektroporationsvorrichtung (1) nach einem der Ansprüche 4-15, wobei mindestens ein Verteilerkanal (70) einen linearen Abschnitt (71) distal zu einem gekrümmten Abschnitt (72) aufweist, sodass der Weg einer elektrotherapeutischen Vorrichtung (60) in ihrer ausgezogenen Position angewendet im Wesentlichen linear ist.

## Revendications

1. Dispositif d'électroporation (1) destiné à induire un champ électrique dans le corps d'un patient, comprenant une pluralité de dispositifs électrothérapeutiques (60) ayant des extrémités distales (64) respectives, chacun des dispositifs électrothérapeutiques (60) étant disposé de façon coulissante par rapport à un point de référence (P) du dispositif d'électroporation (1), entre une position rétractée et une position étendue, ladite extrémité distale (64) de chacun des dispositifs électrothérapeutiques (60) s'étendant en position distale au-delà de la position de ladite extrémité distale (64) dans ladite position rétractée, dans lequel au moins un desdits dispositifs électrothérapeutiques (60) comprend
• un corps principal allongé (63) **caractérisé en ce qu'**il possède un axe longitudinal (B), une extrémité distale (64) et une extrémité proximale (62) ;
• une pointe terminale électriquement conductrice (61) disposée au niveau de l'extrémité distale (64) de ladite partie de corps allongé (63) ; et
• un premier chemin électriquement conducteur (66) s'étendant le long dudit corps principal allongé (63) de ladite extrémité proximale (62) à ladite extrémité distale (64),
ledit corps principal allongé (63) présente une section transversale (A1) sensiblement uniforme le long dudit axe longitudinal (B), dans lequel une section transversale maximale (A2) de ladite pointe terminale (61) dans une direction perpendiculaire audit axe longitudinal (B) est supérieure à la section transversale (A1) de ledit corps principal allongé (63) et dans lequel ladite pointe terminale (61) desdits dispositifs électrothérapeutiques (60) présente une géométrie de surface externe lisse et arrondie.

2. Dispositif d'électroporation (1) selon la revendication 1, comprenant une tige d'introduction allongée (10), ladite tige d'introduction (10) ayant une pointe distale (13) ; ladite pluralité de dispositifs électrothérapeutiques (60) étant disposés de façon coulissante dans ladite tige d'introduction (10) entre une position rétractée, dans laquelle lesdites pointes terminales (61) sont enfermées à l'intérieur de ladite tige d'introduction (10) ou complètement ou partiellement à l'intérieur de ladite pointe distale (13) ou situées sur une surface externe de ladite pointe distale (13), et une position étendue, dans laquelle lesdites pointes terminales (61) s'étendent depuis ladite pointe distale (13) vers une pluralité de positions angulaires espacées s'étendant latéralement.

3. Dispositif d'électroporation (1) selon la revendication 2, dans lequel lesdites pointes terminales (61) desdits dispositifs électrothérapeutiques (60) peuvent être déviées par rapport à un axe longitudinal (L) de ladite tige (10) quand elles sont déployées/étendues en position étendue, de manière à ce qu'au moins une projection planaire prise dans un plan perpendiculaire audit axe longitudinal (L) d'une distance (D1) entre au moins une paire de pointes terminales (61) desdits dispositifs électrothérapeutiques (60) soit supérieure à une étendue maximale (D2) d'une section transversale de ladite tige d'introduction (10), ladite section transversale étant prise dans un plan perpendiculaire audit axe longitudinal (L) au niveau d'une extrémité distale (12) de ladite tige d'introduction (10).

4. Dispositif d'électroporation (1) selon la revendication 3, dans lequel la déviation desdites pointes terminales (61) d'au moins un desdits dispositifs électrothérapeutiques (60), dans leur position étendue, est fournie par une courbure d'un canal de distribution (70) prévu au moins dans ladite pointe distale (13) de la tige d'introduction (10).

5. Dispositif d'électroporation (1) selon l'une quelconque des revendications 2 à 4, dans lequel les pointes terminales (61) sont enfermées à l'intérieur de la pointe distale (13) dans leur position rétractée.

6. Dispositif d'électroporation (1) selon la revendication 5, dans lequel au moins une pointe terminale (61) est cachée à l'intérieur d'un élargissement (73) formé à l'extrémité la plus distale des canaux de distribution (70) prévus dans ladite pointe distale (13), dans leur position rétractée.

7. Dispositif d'électroporation (1) selon l'une quelconque des revendications 2 à 4, dans lequel lesdites pointes terminales (61) sont situées sur une surface externe de ladite pointe distale (13) de ladite tige d'introduction (10) dans la position rétractée et dans lequel la pointe distale est recouverte d'une couche pouvant être dissoute, de sorte que lesdites pointes terminales (61) soient enfermées dans ladite couche pouvant être dissoute, dans la position rétractée, et de sorte que ladite couche pouvant être dissoute présente une forme essentiellement lisse, arrondie, non coupante avec une transition essentiellement lisse, non coupante vers la tige d'introduction (10).

8. Dispositif d'électroporation (1) selon l'une quelconque des revendications 1 à 7, dans lequel ladite pointe terminale (61) et ledit chemin électriquement conducteur (66) sont formés dans des matériaux électriquement conducteurs différents.

9. Dispositif d'électroporation (1) selon l'une quelconque des revendications 1 à 8, dans lequel ladite partie de corps principal allongé (63) constitue ledit premier chemin électriquement conducteur (66).

10. Dispositif d'électroporation (1) selon l'une quelconque des revendications 1 à 9, dans lequel lesdites parties de corps principal allongé (63) comprennent en outre une couche isolante non conductrice extérieure.

11. Dispositif d'électroporation (1) selon la revendication 10, dans lequel au moins l'un des dispositifs électrothérapeutiques (60) a une portion de ladite partie de corps principal allongé (63) qui est non isolée électriquement afin de comprendre une seconde surface électriquement conductrice (161).

12. Dispositif d'électroporation (1) selon la revendication 11, dans lequel la ou lesdites secondes surfaces électriquement conductrices (161) comprennent un point à une distance (d3) de et dans une direction perpendiculaire audit axe longitudinal central (B) supérieure à la distance maximale (d2) d'un quelconque point sur la surface externe de ladite partie de corps allongé et dans une direction perpendiculaire audit axe longitudinal central (d2).

13. Dispositif d'électroporation (1) selon l'une quelconque des revendications 1 à 12, dans lequel au moins une partie de corps principal allongé (63) d'un dispositif électrothérapeutique (60) comprend un passage de fluide (68) s'étendant dans la direction dudit axe longitudinal (B) au travers duquel une dose de molécules thérapeutiques peut être administrée depuis un réservoir relié ou pouvant être relié à l'extrémité proximale (62) dudit dispositif électrothérapeutique (60).

14. Dispositif d'électroporation (1) selon l'une quelconque des revendications 1 à 13, dans lequel les corps principaux (63) des dispositifs électrothérapeutiques (60) présentent une forme de section sensiblement circulaire, et dans lequel le diamètre desdits corps principaux (63) est inférieur à 2 mm, de préférence inférieur à 1 mm, plus préférablement inférieur à 0,5 mm et encore plus préférablement inférieur à 0,2 mm.

15. Dispositif d'électroporation (1) selon l'une quelconque des revendications 1 à 14, dans lequel les pointes terminales (61) des dispositifs électrothérapeutiques (60) présentent une extension transversale maximale dans une direction perpendiculaire audit axe (B) inférieure à 3 mm, de préférence inférieure à 1,5 mm, plus préférablement inférieure à 0,75 mm et encore plus préférablement inférieure à 0,3 mm.

16. Dispositif d'électroporation (1) selon l'une quelconque des revendications 4 à 15, dans lequel au moins un canal de distribution (70) a une section linéaire placée en position distale par rapport à une section courbe (72) de sorte que le chemin d'un dispositif électrothérapeutique (60) déployé dans sa position étendue soit sensiblement linéaire.
